(19) Europäisches Patentamt
European Patent Office
Office européen des brevets

(11) EP 1 017 800 B1

(12) # EUROPEAN PATENT SPECIFICATION

(45) Date of publication and mention of the grant of the patent:
**08.11.2006 Bulletin 2006/45**

(21) Application number: **98942944.4**

(22) Date of filing: **17.09.1998**

(51) Int Cl.:
***C12N 15/11*** *(2006.01)* ***C07H 21/02*** *(2006.01)*
***A61K 31/70*** *(2006.01)* ***A61K 31/505*** *(2006.01)*
*C07D 239/95* *(2006.01)* *C12N 9/10* *(2006.01)*

(86) International application number:
**PCT/GB1998/002820**

(87) International publication number:
**WO 1999/015648 (01.04.1999 Gazette 1999/13)**

(54) **ANTISENSE OLIGONUCLEOTIDES AGAINST THYMIDYLATE SYNTHASE**

ANTISENSE-OLIGONUKLEOTIDE GEGEN THYMIDYLATSYNTHASE

OLIGONUCLEOTIDES ANTISENS DIRIGES CONTRE LA THYMIDYLATE SYNTHASE

(84) Designated Contracting States:
**AT BE CH CY DE DK ES FI FR GB GR IE IT LI LU MC NL PT SE**

(30) Priority: **23.09.1997 GB 9720107**
**17.10.1997 GB 9722012**
**06.06.1998 GB 9812140**

(43) Date of publication of application:
**12.07.2000 Bulletin 2000/28**

(73) Proprietors:
- **Sarissa, Inc.**
  **London, Ontario N6A 4L6 (CA)**
- **ISIS PHARMACEUTICALS, INC.**
  **Carlsbad, CA 92008 (US)**

(72) Inventors:
- **KOROPATNICK, Donald James,**
  **London Reg.Cancer Centr**
  **London, Ontario N6A 4L6 (CA)**
- **VINCENT, Mark, David,**
  **London Reg. Cancer Centre**
  **London, Ontario N6A 4L6 (CA)**
- **DEAN, Nicholas, Mark,**
  **Isis Pharmaceuticals**
  **Carlsbad, CA 92008 (US)**

(74) Representative: **Dunleavy, Kevin James et al**
**Knoble & Yoshida LLC,**
**p/o De Vries & Metman,**
**Overschiestraat 180**
**1062 XK Amsterdam (NL)**

(56) References cited:
**EP-A- 0 290 144** **WO-A-98/33518**
**WO-A-98/49287**

- **JU J.: "INCREASED DRUG SENSITIVITY OF TUMOR CELLS BY MANIPULATION OF CELL CYCLE CONTROL GENES" 1996 , THESIS UNIVERSITY OF SOUTHERN CALIFORNIA, PAGE (S) 100 - 130 XP002081365 see the whole document**
- **FERGUSON P.J. ET AL: "Enhancement of Tomudex cytotoxicity by an antisense oligonucleotide against thymidylate synthase mRNA". PROCEEDINGS OF THE 89TH ANNUAL MEETING OF THE AMERICAN ASSOCIATION FOR CANCER RESEARCH, NEW ORLEANS, MARCH 28-APRIL 1, 1998, VOL. 39, PAGE 416, ABSTRACT 2831 XP002094749**
- **KOROPATNICK J. ET AL: 'Discrimination of run-on transcription from constitutive genes and antisense expression vectors in the same cells' BIOTECHNIQUES vol. 22, no. 1, January 1997, pages 64 - 66, XP002081368**
- **DATABASE EMBL-EBI [Online] HUMAN MRNA FOR THYMIDYLATE SYNTHASE EC 2.1.1.45, 12 September 1993 TAKEISHI K. ET AL: 'Nucleotide sequence of a functional cDNA for human thymidylate synthase' Database accession no. X02308**
- **TAKEISHI K. ET AL: 'Nucleotide sequence of a functional cDNA for human thymidylate synthase' NUCLEIC ACIDS RESEARCH vol. 13, no. 6, 1985, pages 2035 - 2043, XP002090130**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 017 800 B1

Printed by Jouve, 75001 PARIS (FR)

## Description

This invention relates to antisense oligodeoxynucleotides targeted to sequences in thymidylate synthase (TS) mRNA. In particular the invention relates to antisense oligodeoxynucleotides targeted to sequences in the 3' end of TS mRNA, which antisense oligodeoxynucleotides are both cytostatic on their own when administered to human tumour cell lines, and also enhance the toxicity of the anticancer drugs such as Tomudex™ administered to those cells. In contrast, antisense oligodeoxynucleotides targeted to sequences at or near the translation start site at the 5' end of TS mRNA have either no effect, or enhance cell growth, when administered on their own. In addition, antisense nucleic acids targeted to these 5' sequences (but not to 3' sequences) induce TS gene transcription. The invention also relates to a combination product comprising an antisense oligodeoxynucleotide in combination with an anticancer agent such as Tomudex™ (N-(5-[N-(3,4-dihydro-2-methyl-4-oxoquinazolin-6-ylmethyl)-N-methylamino]-2-thenoyl)-L-glutamic acid) or the Zeneca™ development compound ZD 9331 ((S)-2-(2-fluoro-4-[N-(4-hydroxy-2,7-dimethylquinazolin-6-ylmethyl)-N-(prop-2-ynyl)amino]benzamido)-4-(1H-1,2,3,4-tetrazol-5-yl)butyric acid), and to the use of such a combination product in the treatment of cancer.

Thymidylate synthase (TS) (EC 2.1.1.45) catalyses the conversion of deoxyuridylate to thymidylate, and is a housekeeping enzyme essential for the only intracellular *de novo* synthesis of thymidylate (Danenberg, 1977). TS gene expression is tightly regulated with respect to cell proliferation state (Maley and Maley, 1960; Locksin et al, 1979). As such, the TS gene is part of a group of genes whose expression is elevated at the $G_i$/S cell cycle boundary, and it has been suggested that transcription of several S-phase genes (including dihydrofolate reductase and thymidine kinase) is controlled in part by the E2F family of transcription factors (Farnham *et al.,* 1993; Mudrak *et al.,* 1994). In fact, transfection of active E2F1 genes into mouse cells induces expression of TS and other S-phase and cell cycle-regulated genes (De Gregori *et al.,* 1995). As cells progress through the cell cycle from $G_o$ through S phase, TS mRNA levels increase approximately 20-fold and TS enzyme activity increases about 10-fold (Navalgund *et al.,* 1980). However, TS gene transcription rate is upregulated only 2 to 4 times, suggesting that post-transcriptional events play a major role in TS regulation (Ayusawa *et al.,* 1986; Jenh *et al.,* 1985; Johnson, 1994). Differences in TS mRNA stability are not likely to be critical in regulation, as TS mRNA half-life is about 8 hours in both resting and growing rodent cells (Jenh *et al.,* 1985). On the other hand, TS mRNA translation appears to be regulated by the TS protein itself, which specifically interacts at two sites within its own mRNA to inhibit protein production (Cbu *et al.,* 1991, 1993b, 1994; Voeller *et al.,* 1995). Translation of other mRNAs (including c-*myc* mRNA) may also be regulated by interactions with TS protein (Chu *et al.,* 1995).

Because of its role in DNA precursor synthesis, TS has been identified as a potential target for cancer chemotherapeutic agents (Hardy *et al.,* 1987). High TS levels have been correlated with poor prognosis in patients with ovarian cancer (Suzuki *et al.,* 1994), rectal cancer (Johnson, 1994) childhood acute non-lymphoblastic leukaemia (Volm *et al.,* 1994), and non-small cell lung carcinoma (Volm and Mattern, 1992). However, its prognostic value is not high in all tumour types (Peters *et al.,* 1986, 1994). Two types of TS inhibitors have been developed: (a) nucleotide analogues (including 5-FU, its riboside, and deoxyriboside derivatives) which must be activated to 5-fluorodeoxyuridylate (FdUMP) within cells to be effective (Heidelberger *et al.,* 1983) and (b) 5,10-$CH_2FH_4$ (antifolate) analogues, including *N*-10-propargyl-5,8-dideazafolate (CB3717) (Calvert *et al.,* 1986) and Tomudex™ (ZD 1694; *N*-[5-(*N*-[3,4-dihydro-2-methyl-4-oxoquinalin-6-ylmethyl]-*N*-methylamino)-2-thenoyl]-L-glutamic acid) (Jackman *et al.,* 1991a, 1991b). Although. Tomudex™ and 5-FU inhibit TS and have potent cytotoxic and antitumour activity (Heidelberger *et al.,* 1983; Keyomarsi *et al.,* 1993), they have an unusual biochemical effect. When human cancer cell lines are treated with 5-FU or Tomudex™, TS levels increase rapidly, perhaps as a result of the release of translational inhibition by the TS protein (Keyomarsi *et al.,* 1993; Chu *et al.,* 1990; Chu *et al.,* 1993a).

It has been speculated that the release of translational inhibition that accompanies binding and inactivation of TS by chemotherapeutic agents (including Tomudex™ and 5-FU) might be prevented by treating cells with agents that could replace the specific interaction between TS mRNA and TS protein, and inhibit translation (Keyomarsi *et al.,* 1993) but no such agents were described. In another speculative article it was hypothesised that antisense nucleic acids designed to both reduce the ability of TS mRNA to direct protein production, and to interact with the TS protein binding site, may be useful in complementing the effectiveness of drugs targeted against TS (Rapaport *et al.,* 1992).

In Ju, J. (Ju, J.: "Increased Drug Sensitivity of Tumor Cells by Manipulation of Cell Cycle Control Genes, 1996, Thesis University of Southern California, pages 100-130), antisense oligonucleotides targeted to the translational initiation site of thymidylate synthase (TS) mRNA have been used to inhibit translation in an *in vitro* translation system, however, treatment of human colon cancer HT-29 cells with antisense oligodeoxynucleotides resulted in an increase in the amount of TS in the cells and decreased their sensitivity to 5-fluoro-2'-deoxyuridine (FdUrd). By contrast, a plasmid construct containing a TS antisense gene fragment (+1 to +422) used to produce an antisense product taken from the same region as the antisense oligonucleotides tested was shown to reduce expression of TS protein and to enhance the sensitivity of these cells to FdUrd.

In previous patent applications, GB 9720107.3 and GB 9722012.3, we disclosed how to specifically down-regulate the expression of TS in human breast cancer (MCF-7) cells in two ways. First, we both transiently and stably transfected the cells with vectors expressing antisense RNA molecules directed to hybridise to three different regions of the TS mRNA.

Targeted sequences were: (1) sequences participating in the formation of a putative stem- loop structure surrounding the translation start site, and immediately adjacent and 3'to that site (these sequences also participate in binding TS protein to modulate translation), (2) the exonl/exon2 boundary and (3) the 3' end of the mature cytoplasmic mRNA. Antisense TS RNA was expressed from these vectors (as assessed by northern blot analysis and a novel modification of the run-on transcription assay to measure antisense transcription against background constitutive TS gene expression) (Koropatnick et al., 1997). Second, we transiently transfected cells with single-stranded oligodeoxynucleotides targeted to hybridise to: (a) the translation start site and sequences surrounding it, (b) a sequence proximal to the translation start site and participating in the putative stem-loop structure, and (c) the translation stop site near the 3'end of the mature cytoplasmic RNA.

The present invention is based on our discovery that an antisense oligonucleotide, oligo 83, complementary to a sequence in the TS mRNA 3' untranslated region, down- regulated the level of TS mRNA and protein, inhibited cell proliferation and enhanced the cytotoxicity of TS-directed chemotherapy drugs.

In a first aspect of the invention we provide an antisense oligodeoxynucleotide which hybridises to a target nucleic acid sequence in thymidylate synthase and which selectively inhibits thymidylate synthase production in mammalian cells and is nuclease resistant. The oligonucleotide consists of 8-50 nucleotides and includes a nucleotide sequence complementary to a sequence at or near the translational stop site at the 3' end of the TS gene, which sequences lie in the region between bases 800 and 1536, using the sequence numbering described for human thymidylate synthase mRNA (cDNA) by Takeishi et al., 1985. More preferably the sequences lie in the region between bases 1000 and 1530. Most preferably the sequences lie in the region between bases 1030 and 1460.

An antisense oligodeoxynucleotide is an oligonucleotide which is designed to hybridise to a specific region of a target nucleic acid sequence. The target nucleic acid is the TS gene or mRNA transcribed from the TS gene. Preferably the target nucleic acid is the mRNA encoding thymidylate synthase.

The effects of antisense oligonucleotides on thymidylate synthase expression can be measured using procedures which are well known to persons skilled in the art. In the present application, effects on mRNA levels have been measured by Northern blot analysis and nuclear run-on transcription assay, and effects on the growth of human tumour cells have been measured by counting cell numbers using a Coulter™ counter.

Antisense oligonucleotides to thymidylate synthase may inhibit, stimulate or have no effect on thymidylate synthase expression. Of these, preferred antisense oligonucleotides are those which inhibit thymidylate synthase expression.

By inhibition of thymidylate synthase expression we mean inhibition of at least 10% relative to the untreated control, measured at day 4 using the assay described in Example 1.2.

Preferably inhibition of thymidylate synthase expression is at least 20% and most preferably inhibition is at least 40%.

Preferably, the antisense oligonucleotides are from about 8 to about 50 nucleotides in length, more preferably from about 12 to about 40 nucleotides in length and most preferably from about 16 to about 30 nucleotides in length.

Specific examples of sequences of antisense oligonucleotides which regulate thymidylate synthase activity are shown in Table 1. Sequences of antisense oligonucleotides that inhibit thymidylate synthase activity in accordance with the invention shown in Table 1 are SEQ ID NOS. 83 and 86. The regions of TS mRNA targeted by the oligonucleotides are shown in Figure 7.

*Table 1:*

| ANTISENSE OLIGONUCLEOTIDE | SEQUENCE |
|---|---|
| OLIGO 83 | GCCAGTGGCAACATCCTTAA |
| OLIGO 86 | AAGCACCCTAAACAGCCATT |
| OLIGO 90 | GCAGCTCCGAGCCGGCCACA |
| OLIGO 91 | GCCGGCCACAGGCATGGCGC |
| OLIGO 92 | GCCGGCCACAGGCATGGCGC |
| OLIGO 93 | GGCATGGCGCGGCGGGCGGG |
| ODN 32 | ATGCGCCAACGGTTCCTAAA |
| PAS/TSS | UGUGGCCGGCUCGGAGCUGCCGCGCCGGCC |

(continued)

| ANTISENSE OLIGONUCLEOTIDE | SEQUENCE |
|---|---|
| PAS/EXON1,2 | GCUACAGCCUGAGAGAUGAAUUCCCUCUGC |

It will be appreciated that the invention is not restricted merely to those specific antisense oligonucleotides 83 and 86 which inhibit thymidylate synthase production that are disclosed in Table 1 above but encompasses oligonucleotides of from about 8 to about 50 nucleotides in length which selectively inhibit thymidylate synthase production and which are selected from those regions of the TS gene which are described hereinbefore.

Hybridisation of an antisense oligonucleotide to its target nucleic acid sequence is mediated by the formation of hydrogen bonds between complementary bases on each nucleic acid strand. Hybridisation may occur between nucleic acid strands which have varying degrees of complementarity, depending on the hybridisation conditions employed. The term "specifically hybridisable"is used to describe an oligonucleotide which has a sufficient degree of complementarity to ensure stable, specific binding to its target sequence, whilst avoiding non-specific binding to non-target sequences.

Antisense oligonucleotides may be designed to hybridise to any region within the thymidylate synthase mRNA molecule, including the coding region, the 5' untranslated region, the 3' untranslated region, the 5' cap region, introns and intron/exon splice junctions.

Hybridisation of the antisense oligonucleotide to thymidylate synthase mRNA may affect any aspect of mRNA function, for example mRNA translocation, mRNA splicing, mRNA translation, or the feedback inhibition mechanism regulated by the binding of thymidylate synthase protein to binding sites within the thymidylate synthase mRNA molecule.

An oligonucleotide is a polymeric molecule which is assembled from nucleotide or nucleoside monomers. The monomers may consist of naturally occurring bases, sugars and inter-sugar linkages or may also contain non-naturally occurring derivatives which modify the properties of the oligonucleotide, for example, phosphorothiorated oligonucleotides have been used in the present application to increase resistance to nuclease degradation.

Preferred oligonucleotides may contain phosphorothiorates, phosphotriesters, methyl phosphonates or short chain alkyl, cycloalkyl or heteroatomic intersugar linkages. Other preferred oligonucleotides may be methoxy-ethoxy winged or may contain a peptide nucleic acid backbone. Particularly preferred oligonucleotides are those containing phosphorothiorates (Summerton, J.E. and Weller, D.D., U.S. Patent No: 5,034,506).

The oligonucleotides may be manufactured using any convenient method of synthesis. Examples of such methods may be found in standard textbooks, for example "Protocols for Oligonucleotides and Analogues; Synthesis and Properties," Methods in Molecular Biology Series; Volume 20; Ed. Sudhir Agrawal, Humana ISBN: 0-89603-247-7; 1993; 1st Edition.

In a further aspect of the invention, there is provided a pharmaceutical composition comprising an antisense oligonucleotide targeted to thymidylate synthase as defined hereinbefore in a pharmaceutically acceptable diluent or carrier.

There is provided a method for the treatment of cancer (or a method for providing an antiproliferative effect) which comprises administering to a warm-blooded animal an effective amount of an oligonucleotide targeted to thymidylate synthase as defined hereinbefore which does not form part of the invention, as claimed. The invention also provides the use of such an oligonucleotide in the production of a new medicament for the treatment of cancer ( or for the treatment of proliferative disease.

In a further aspect of the present invention, there is provided a combination product comprising an antisense oligonucleotide targeted to thymidylate synthase in combination with an anticancer agent. The antisense oligonucleotide and the anticancer agent may be administered separately, sequentially, simultaneously or in a mixture.

The anticancer agent may cover three main categories of therapeutic agent:

(i) thymidylate synthase inhibitors such as Tomudex™ (N-(5-[N-(3,4-dihydro-2-methyl-4-oxoquinazolin-6-ylmethyl)-N-methylamino]-2-thenoyl)-L-glutamic acid) (European Patent Application no. 0239362, Example 7, compound no. 8 therein); Zeneca™ development compound ZD9331 ((S)-2-(2-fluoro-4-[N-(4-hydroxy-2,7-dimethylquinazolin-6-ylmethyl)-N-(prop-2-ynyl)amino]benzamido)-4-(1H-1,2,3,4-tetrazol-5-yl)butyric acid) (European Patent Application no. 0562734, Example 3 thereof); LY 231514 (Eli Lilly Research Labs, Indianapolis, IN); 1843U89 (Glaxo-Wellcome, Research Triangle Park, NC); AG337 and AG331 (both by Agouron, La Jolla, CA) (Touroutoglou and Pazdur, Clin. Cancer Res., 2, 227-243,1996).

(ii) cytostatic agents such as antioestrogens (for example tamoxifen, toremifene, raloxifene, droloxifene, iodoxyfene), progestogens (for example megestrol acetate), aromatase inhibitors (for example anastrozole, letrazole, vorazole, exemestane), antiprogestogens, antiandrogens (for example flutamide, nilutamide, bicalutamide, cyproterone acetate), LHRH agonists and antagonists (for example goserelin acetate, luprolide), inhibitors of testosterone 5α-dihydroreductase (for example finasteride), anti-invasion agents (for example metalloproteinase inhibitors like marimastat and inhibitors of urokinase plasminogen activator receptor function) and inhibitors of growth factor function, (such growth factors include for example EGF, FGFs, platelet derived growth factor and hepatocyte growth factor such inhibitors include growth factor antibodies, growth factor receptor antibodies, tyrosine kinase inhibitors and serine/threonine kinase inhibitors).

(iii) antiproliferative/antineoplastic drugs and combinations thereof, as used in medical oncology, such as antimetabolites (for example antifolates like methotrexate, fluoropyrimidines like 5-fluorouracil, FUdR, ftorafur, FdUR, purine and adenosine analogues, cytosine arabinoside); antitumour antibiotics (for example anthracyclines like doxorubicin, daunomycin, epirubicin and idarubicin;) mitomycin-C, dactinomycin, mithramycin; platinum derivatives (for example cisplatin, carboplatin, oxaliplatin); alkylating agents (for example nitrogen mustard, melphalan, chlorambucil, busulphan, cyclophosphamide, ifosfamide, nitrosoureas, thiotepa); antimitotic agents (for example vinca alkaloids like vincrisitine and taxoids like taxol, taxotere); topoisomerase inhibitors (for example epipodophyllotoxins like etoposide and teniposide, amsacrine, topotecan).

The anticancer treatment may also be radiotherapy.

Particularly preferred anticancer agents are thymidylate synthase inhibitors such as Tomudex™(N-(5-[N-(3,4-dihydro-2-methyl-4-oxoquinazolin-6-ylmethyl)-N-methylamino]-2-thenoyl)-L-glutamic acid) (European Patent Application no. 0239362, Example 7, compound no. 8 therein) and the Zeneca™ development compound ZD9331 ((S)-2-(2-fluoro-4-[N-(4-hydroxy-2,7-dimethylquinazolin-6-ylmethyl)-N-(prop-2-ynyl)amino]benzamido)-4-(1H-1,2,3,4-tetrazol-5-yl)butyric acid) (European Patent Application no. 0562734, Example 3 thereof).

In a further aspect of the present invention there is provided a pharmaceutical composition comprising a combination product as defined hereinbefore and a pharmaceutically acceptable diluent or carrier.

Any pharmaceutical composition as defined hereinbefore may be in a form suitable for oral use, for example a tablet, capsule, aqueous or oily solution, suspension or emulsion; for topical use, for example a cream, ointment, gel or aqueous or oily solution or suspension; for nasal use, for example a snuff, nasal spray or nasal drops; for vaginal or rectal use, for example a suppository; for administration by inhalation, for example as a finely divided powder such as a dry powder, a microcrystalline form or a liquid aerosol; for sub-lingual or buccal use, for example a tablet or capsule; or particularly for parenteral use (including intravenous, subcutaneous, intramuscular, intravascular or infusion), for example a sterile aqueous or oily solution or suspension. In general the above compositions may be prepared in a conventional manner using conventional excipients.

Tomudex™ is conveniently administered to humans by intravenous injection of a sterile aqueous solution at a dose in the range, for example, of 1 to 4 $mg/m^2$ of body surface area once every three weeks, preferably at a dose of 3 $mg/m^2$ once every three weeks.

ZD 9331 is conveniently dosed to humans by oral administration of a solid dosage form or by intravenous injection of a sterile aqueous solution. The oral dosage form is conveniently administered to humans at a total dose in the range, for example, of about 1 to 100 mg/kg (i.e. about 35 $mg/m^2$ to 3.5 $g/m^2$) every three weeks given by a continuous or an intermittent dosing schedule, for example a dosing schedule of a three week dosing cycle comprising daily doses on days 1 to 5 only followed by no further doses until the next dosing cycle or a dosing schedule of a four week dosing cycle comprising daily doses on days 1 to 14 only followed by no further doses until the next dosing cycle. Preferably the oral dosage form is administered to humans at a total dose in the range, for example, of about 1 to 30 mg/kg every three or four week dosing cycle. The sterile aqueous solution is conveniently administered intravenously to humans at a total dose of up to 100 $mg/m^2$ every three weeks given by a continuous or an intermittent dosing schedule, for example, a dosing schedule of one dose per three week dosing cycle, a dosing schedule of a three week dosing cycle comprising daily doses on days 1 to 5 only followed by no further doses until the next dosing cycle, a dosing schedule of a three week dosing cycle comprising doses on days 1 and 8 only followed by no further doses until the next dosing cycle or a dosing schedule of a three week dosing cycle comprising continuous infusion on days 1 to 5 followed by no further dosing until the next dosing cycle. Preferably the sterile aqueous solution is administered intravenously to humans at a total dose in the range, for example, of about 20 to 50 $mg/m^2$ every three weeks given by a continuous or an intermittent dosing schedule as illustrated hereinbefore.

The antisense oligonucleotide is conveniently administered to humans by intravenous injection of a sterile aqueous solution at a dose per dosing cycle in the range, for example, of 0.1 μg to 1 g, preferably at a dose of 1 mg to 100mg.

The amount of active ingredient that is combined with one or more excipients to produce appropriate dosage forms will necessarily vary depending upon the particular component of the combination product, the host treated and the particular route of administration. For example, a formulation intended for oral administration to humans will generally contain, for example, from 0.5μg to 2g of active agent compounded with appropriate and convenient amounts of excipients which may vary from about 5 to about 98 percent by weight of the total composition. A formulation intended for parenteral administration to humans will generally contain 0.1μg to 50mg. Dosage unit forms will generally contain about 1μg to about 500mg of an active ingredient.

There is provided a method for the treatment of cancer (or a method for providing an antiproliferative effect) which comprises administering to a warm-blooded animal an effective amount of a combination product as defined above which does not form part of the invention, as claimed. The invention also provides the use of such a combination product in the production of a new medicament for the treatment of cancer (or for the treatment of proliferative disease).

Abbreviations used in this application are set out below.

| | |
|---|---|
| TS | thymidylate synthase |
| CMV | cytomegalovirus |
| 5-FU | 5-fluorouracil |
| LFA | lipofectamine |
| PBS | phosphate-buffered saline |
| GAPDH | glyceraldehyde-3-phosphate-dehydrogenase |
| FBS | fetal bovine serum |
| MT | metallothionein |
| ODN | oligodeoxynucleotide |
| bp | base pairs |
| DMEM | Dulbecco's modified Eagle medium |
| oligo | oligonucleotide |

The invention will now be illustrated but not limited by reference to the following Example and Figures wherein:

*Figure 1* shows that MCF-7 cell growth is inhibited by transfection with antisense TS oligo 86 (targeted to the translation stop site), but is enhanced by transfection with antisense TS oligos 90 or 92 (targeted to sequences at or near the translation start site).

Cells were transiently-transfected with 0.5 or 1.0 μM antisense TS oligonucleotides in Lipofectin™ as described. Cell numbers were measured by Coulter™ counter in triplicate flasks after 4 days of growth. Control cells were treated with Lipofectin without oligonucleotides. Cell growth is expressed as a percentage of growth of control cells.
*: Significantly higher than control ($p<0.05$, one way analysis of variants).
**: Significantly lower than control ($p<0.05$, one way analysis of variants).

*Figure 2* shows that HeLa cell growth is inhibited by transfection with antisense TS oligo 86 (targeted to the translation stop site), but is enhanced after transfection with antisense TS oligo 91 (targeted to the translation start site).

HeLa cells were transfected with 0.05 or 0.10 μM antisense TS oligonucleotides in Lipofectin™ (10 μg/ml) for 4 hours as described. Note that oligo concentrations are considerably lower than those used for MCF-7 cells. The efficiency of Lipofectin™-mediated DNA transfection of HeLa cells is greater than for MCF-7 cells. Lipofectin™ was removed, the cells were trypsinised, and 25,000 viable cells per flask were plated in tissue culture flasks. Cell numbers were measured by Coulter™ counter in triplicate flasks after 4, 7 and 8 days of growth. Control cells were treated with Lipofectin™ without oligonucleotides. Cell growth is expressed as a percentage of growth of control cells.
*: Significantly higher than control ($p<0.05$, Student's t-test).
**: Significantly lower than control (Student's t-test).

*Figure 3* shows that HeLa cell growth is inhibited by transfection with antisense TS oligo 83 (targeted to a 3' untranslated sequence downstream of the translation stop site), but it is not affected by transfection with antisense TS oligo 81 (targeted to a sequence in the 3' untranslated region of TS mRNA). The experimental protocol was as described in the legend to Figure 2.

*Figure 4* shows that transient transfection of HeLa cells with oligo 86 (targeted to the TS translation stop site) enhances sensitivity to Tomudex™ and that oligo 91 (targeted to the TS translation start site) reduces sensitivity to Tomudex™.

HeLa cells were transfected with 0.05 and 0.10 μM antisense TS oligonucleotides and plated in flasks at low density, as described for Figure 2. Tomudex (0-8 nM) was added (triplicate flasks for each Tomudex™ concentration) and the cells allowed to proliferate for 7 days. Cell numbers were measured by Coulter™ counting at that time. Survival is plotted as a percentage of growth in celts transfected with oligonucleotide, but untreated with Tomudex™ Therefore, these data reveal inhibition or enhancement of Tomudex™ killing independent of growth inhibition or enhancement induced by oligonucleotides in the absence ofTomudex. The mean of three values is plotted. Error bars were smaller than the size of the symbol in each case.
*: Significantly different from control ($p<0.05$, Student's t-test).

*Figure 5* shows that transient transfection of HeLa cells with oligo 83 (targeted to a sequence in the 3' untranslated region of TS mRNA) enhances sensitivity to Tomudex whereas oligo 81 (targeted to a 3' sequence downstream of that targeted by oligo 83) has no effect on Tomudex™ sensitivity.

HeLa cells were transfected with 0.10 μM antisense TS oligonucleotides and plated in flasks at low density, as described for Figure 2. Tomudex (0-10 nM) was added (triplicate flasks for each Tomudex™concentration) and the cells allowed to proliferate for 4 days. Cell numbers were measured by Coulter™ counting at that time. Survival is plotted as a percentage of growth in cells transfected with oligonucleotide, but untreated with Tomudex™. Therefore, these data reveal enhancement of Tomudex™ killing independent of growth inhibition induced by oligonucleotides in the absence of Tomudex.™ The mean of three values is plotted. Error bars were smaller than the size of the symbol in each case.
*: Significantly different from control ($p<0.05$, Student's t-test).

*Figure 6* shows that antisense TS oligo 91, but not oligo 86, stimulates TS gene transcription in human HeLa cells.

The same HeLa cells for which data are presented in Figure 2 were assessed for run-on transcription of TS, glyceraldehyde phosphate dehydrogenase (GAPDH), and 18S rRNA genes. Briefly, cells were transfected with 0.05 and 0.10 μM antisense TS oligonucleotides in Lipofectin™ (10 μg/ml), or with Lipofectin™alone (LFA control) for 4 hours as described. Lipofectin™ was removed and cells were trypsinised and replated in tissue culture flasks. Four days after transfection, nuclei were isolated from approximately $5x10^6$ cells for each treatment and initiated TS, GAPDH, and 18S rRNA transcripts allowed to incorporate [$^{32}P$]-CTP for 30 minutes. Alcohol-precipitable radiolabeled RNA was hybridised for 48 hours to unlabeled TS, GAPDH, and 18S rRNA cDNA immobilised in triplicate dots on nylon membrane as described. Relative transcription rate is presented as:

$$\text{Relative transcription rate} = \frac{\text{(hybridisation signal from gene of interest)}}{\text{(hybridisation signal for GAPDH or 18S rRNA genes)}}$$

*Figure 7* shows portions of the sequence of human mRNA (cDNA) for thymidylate synthase (EC 2.1.1.45), bases 1 to 1536.

Figure 7a shows the sequence of human mRNA (cDNA) for thymidylate synthase (EC 2.1.1.45) bases 1 to 1536

*Figure 8:* shows that antisense TS ODN 83 inhibits HeLa cell proliferation. HeLa cells transfected with 50 nM antisense TS ODN 83 (•) or 50 nM scrambled control ODN 32 (o) were assessed for cell proliferation at 1, 2, 5, and 6 days following transfection. Data points indicate the average of two measurements, and are representative of qualitatively similar results obtained in 16 independent experiments.

*Figure 9* shows that antisense TS ODN 83 suppresses HeLa cell growth after transfection, followed by recovery to control proliferation rate after 48 hours. HeLa cells were transfected with 50 nM antisense TS ODN 83 or 50 nM scrambled control ODN 32 as described in the legend to Figure 1. Values derived from cells transfected with ODN 32 were normalised to 100%, and each bar indicates the percent of that value measured following treatment with ODN 83 (mean +- SE of 4 independent experiments). Asterisks (*) indicate significant differences ($p<0.02$, Student *t*-test).

*Figures 10* & *11* show that treatment of HeLa cells with ODN 83 leads to decreased TS mRNA levels.

*(Figure 10)* HeLa cells were transfected with ODN 83 or scrambled control ODN 32, or treated with Lipofectamine alone. Cells were harvested at 1, 2, and 4 days post-transfection and total cellular RNA isolated, reverse-transcribed, and TS and GAPDH cDNA amplified by 24 PCR cycles in the same reaction vessel. TS (208 bp) and GAPDH (752 bp) RT/PCR products were confirmed by Southern blotting and hybridisation to specific radioactively-labeled probes.

*(Figure 11)* TS:GAPDH ratio of RT/PCR products from RNA isolated from HeLa cells 1 day after transfection with ODN 83 or ODN 32. Twenty-four, 25, 26, or 27 cycles of PCR amplification were carried out, revealing the same reduction in TS:GAPDH ratio after transfection with ODN 83.

*Figure 12* shows that TS protein levels (inferred by measurements of 5-FdUMP binding) are diminished by antisense TS ODN 83 but not scrambled control ODN 32. 5-FdUMP binding was measured in cells transfected with ODN 83 (hatched bars) or ODN 32 (open bars) at different times following transfection.

(A): Results are plotted as a percent of 5-FdUMP binding in cells transfected with control ODN 32+- SE *(n=5).* The values for ODN 32 (n=5) were normalised to 100% and are shown without error bars.

(B): Results are presented as pmol 5-FdUMP bound per mg total protein (x $10^{-3}$) to reveal that transfection with control ODN 32 had no significant effect on TS protein levels. Error bars indicate errors calculated according to a Student *t*-test, and indicate error due to differences in experimental conditions in 5 measurements taken on different days, and differences due to transfection with different ODNs. The asterisks indicate significant differences (p<0.02) determined by using a paired Student *t*-test.

*Figure 13:* shows that antisense TS ODN 83 sensitises HeLa cells to the toxic effects of 5-FU, 5-FUdR, Tomudex™, and MTX, but not cisplatin or chlorambucil. HeLa cells were transfected with ODN 83 (•) or control ODN 32 (o) and treated with different concentrations of 5-FU (A), 5-FUdR (B), Tomudex™(C), MTX (D), cisplatin (E) or chlorambucil (F) for 4 days, beginning 24 h after transfection. Data points are plotted as the mean +- SE of 4. measurements. Where error bars are not apparent, they are obscured by the symbol. Asterisks (*) indicate significant differences <0.02, Student *t*-test).

## Example 1

### Example 1.1: Experimental Methods

*Cell culture:*

MCF-7 (human breast adenocarcinoma) cells were cultured in DMEM supplemented with 10% fetal bovine serum (FBS), 2 mM glutamine, 10 mM Hepes (pH 7.4) and 0.1% Gentamycin.

*Vector construction:*

Expression vectors pAS/TSS and pAS/exonl,2 were designed to produce, upon transfection into MCF-7 cells, single-stranded antisense RNA molecules containing double-stranded 30 bp oligonucleotides complementary to the TS mRNA at one of two sites. Oligodeoxynucleotides corresponding to each strand of the human TS cDNA at positions 111 to 140 (pAS/TSS; targeting a 30 bp region adjacent to, and 2 bp away from, the translation start site) or 296 to 325 (pAS/exon1,2; targeting a 30 bp region spanning the exon 1/exon 2 boundary) were synthesized. Numbering of bases was according to GenBank™ Accession No. X02308 (Takeishi *et al.,* 1985). In order to facilitate cloning, six additional nucleotides were incorporated (5 at the 5' end, 1 at the 3' end) of each oligodeoxynucleotide to produce *Hind* III or *Xba* I sticky ends when complementary strands were annealed. Single-stranded oligonucleotides (4 mg each) were annealed in 3X SET (450 mM Nad, 60 mM Tris-HCl, 3 mM EDTA, pH 7.8) by treating the mixture for 5 min at 90°C, 5 h at 50°C, then 16 h at 25°C. Double-stranded products were identified by gel electrophoresis, and directionally inserted into the *Hind* III and *Xba* I sites of pRC/CMV (Invitrogen Corp., San Diego; CA). The orientation of cloning was confirmed by direct sequencing.

*Oligodeoxynucleotides:*

Fully phosphorothioated 20-mer oligonucleotides (ODNs) were synthesized by Isis Pharmaceuticals (Carlsbad, CA). The 6 terminal nucleotides at the 5' and 3' ends of each ODN were 2'-methoxyethoxy-modified to make them resistant to intracellular nucleases and increase their stability within cells (M'Kay *et al.,* 1996), but the internal 8 nucleotides without methoxyethoxy groups were susceptible to RNase H cleavage. Oligo 86 was complementary to TS mRNA from base positions 1035 to 1054 (GenBank™ Accession No. X02308; Takeishi *et al.,* 1985), which surround the TS mRNA translation stop site (UAG at bases 1045 to 1047). Oligo 90 was complementary to base positions 111 to 130, which are 3' and proximal to the translation start site (AUG at bases 106 to 109). Oligo 92 was complementary to base positions 101 to 120, including and surrounding the translation start site.

*Transfection:*

Antisense RNA expression vectors were transfected into MCF-7 cells using Lipofectamine (GIBCO BRL, Burlington, ON, Canada), a polycationic liposome formulation. 1.5 X $10^6$ cells were dispensed into 100 X 15 mm tissue culture plates and allowed to adhere overnight. Cells were washed once with 3 ml OptiMEM (GIBCO BRL, Burlington, ON, Canada), followed by exposure to 6 ml of a mixture of double-stranded expression vector DNA (0.83 μg/ml) plus Lipofectamine (1.67 μg/ml) in opti-MEM for 6 h at 37°C in a 5% $CO_2$ incubator. The DNA/Lipofectamine mixture was removed and replaced with 10 ml DMEM with supplements. Control cells were transfected with pRC/CMV vector without insert. Transiently-transfected cells were used within 1 to 6 days. Stably-transfected cells were allowed to recover in complete non-selective medium without Geneticin for 48 h, then grown in the presence of Geneticin (400 μg per ml, active form [GibcoBRL]) to allow selection of colonies harboring pRC/CMV control vectors, or pRC/CMV vectors expressing RNA complementary to sequences surrounding the translation start site (pAS/TSS).

Single-stranded oligodeoxynucleotides (0.5 μM or 1.0 μM) were transiently transfected into 1.5 X $10^6$ MCF-7 cells adhering to 100 X 15 mm tissue culture plates, in a total volume of 5 ml in the presence of Lipofectamine (2 μg/ml) in Opti-MEM. Control cells were treated with

Lipofectamine/Opti-MEM without added DNA. Cells were washed after 6 h, medium plus supplements added as described above, and then grown for 48 hours before isolation of nuclei for run-on transcription measurement.

*Southern blot analysis:*

Total DNA was isolated from pAS/TSS transfected cells as follows. Cells were incubated in lysis buffer (50 mM Tris-HCl [pH8.0]. 100 mM EDTA, 100 mM NaCl, 1% sodium dodecyl sulfate, 0.5 mg/ml proteinase K) for 6 h at 55°C. One-third the volume of 6 M NaCl was added to precipitate non-nucleic acids by centrifugation at 10,000 X *g* for 15 min. The DNA in the supernatant was precipitated in isopropanol and washed with 70% ethanol. DNA was cleaved with *Hind* III for 16 h and analysed by Southern blotting (Sambrook *et al.,* 1989). Blots were hybridized with an [$\alpha$-$^{32}$P]dCTP random primer-labeled pAS/TSS probe (Church and Gilbert, 1984), and were exposed to a phosphor screen and quantitated using a PhosphorImager™ and the ImageQuant™ program (Molecular Dynamics, Sunnyvale, CA). The nylon membranes were stripped and rehybridized with an *Alu* probe (300 bp of a human *Alu* restriction fragment inserted into pBR322 [Jelinek *et al.,* 1980]) in order to quantitate the amount of human DNA loaded in each lane (Koropatnick *et al,* 1988).

*Northern blot analysis:*

RNA was isolated using RNeasy™ columns (Qiagen Inc., Chatsworth, CA) from cells transfected with the pAS/TSS expression vector. Ten or 15 ug of RNA per lane were separated on a 1.4% formaldehyde gel (Sambrook *et al.,*1989) and transferred to a Hybond™-N nylon membrane. Membranes were hybridized (Church and Gilbert, 1984) with either a pAS/TSS-generated riboprobe (Promega Corp., Madison, WI) designed to bind to antisense RNA, or a random primer-labeled, 1.9 kb *Xho* I fragment from pcHTS-1 (a eukaryotic expression vector containing the human TS cDNA: a generous gift from Dr. K. Takeishi, University of Shizuoka, Shizuoka, Japan)(Takeishi *et al.,* 1985). Blots were stripped and rehybridized with cDNA probes to detect 18S ribosomal RNA or glyceraldehyde-3-phosphate dehydrogenase (GAPDH). Images were quantitated using a PhosphorImager™ (Molecular Dynamics, Sunnyvale, CA).

*Isolation of nuclei*:

Relative transcription rates were determined by a nuclear run-on assay (Koropatnick *et al.,* 1997), a modification of the methods of Kikuchi *et al.* (1992) and Almendral *et al.* (1988). Nascent transcripts were extended *in vitro* (Marzluff and Huang, 1984) in parallel reactions using MCF-7 cell nuclei isolated 48 h following transient transfection with control or antisense TS RNA expression vectors, or single-stranded antisense TS oligodeoxynucleotides (or Lipofectamine alone), and from cells stably transfected with antisense RNA expression vectors. Adherent cells were rinsed twice with ice-cold PBS, scraped off with a rubber policeman, pelleted in PBS (5 min, 500 X *g)* and lysed by incubating 5 min at 4° C in 4 ml of lysis buffer (10 mM Tris-Cl, pH 7.4, 10 mM NaCl, 3 mM $MgCl_2$, 0.5% NP-40). All subsequent steps were carried out at 4° C. Complete cell lysis and integrity of released nuclei was checked by light microscopy, and nuclei were pelleted by centrifugation at 500Xg for 5 min. Nuclei were then resuspended in 4 ml of lysis buffer by vortexing, pelleted by centrifugation, resuspended in 200 μl of nuclei storage buffer (40% glycerol, 5 mM $MgCl_2$, 50 mM Tris-HCl [pH 8.0], 0.1 mM FDTA) in a 15 ml conical polypropylene centrifuge tube, and immediately frozen in liquid nitrogen and stored at -120° C until use up to one month later:

*Run-on transcription:*

RNA elongation reactions were performed for 30 min at 30°C using 2 X $10^7$ nuclei/400 μl reaction. Reaction mixtures were composed of 200 μl nuclei storage buffer plus 200 μl of sterile 2X reaction buffer (10 mM Tris-HCl [pH 8.0], 5 mM $MgCl_2$, 0.3 M KCl, 1 mM ATP, 1 mM CTP, mM GTP, 5 mM dithiothreitol, and 2 μl [$\alpha$-$^{32}$P]UTP or [$\alpha$-$^{32}$P]CTP [≈ 3000 Ci/mmole, 10 mCi/ml]). Nucleotides, radionucleotides, and dithiothreitol were added immediately prior to use. Nascent RNA transcripts were allowed to elongate for 30 min at 30°C on a shaking platform, followed by addition of 600 μl of RNase-free DNase I (0.04 units RQ1 DNase I [RNase-free; Promega Corp.], 0.5 M NaCl, 50 mM $MgCl_2$, 2 mM $CaCl_2$, 10 mM Tris-HCl [pH 7.4]). The $^{32}$P-labeled RNA was isolated using Trizol™ (Gibco/BRL), and the final precipitated RNA was dissolved in Church hybridization buffer (1 mM EDTA, 0.5 M $NaHPO_4$, [pH 7.2], 7% sodium lauryl sulphate [SLS]), to a final concentration of 4 X $10^6$ cpm per ml .

*Hybridization of radiolabeled RNA to immobilized unlabeled probes:*

In order to distinguish between TS sense and antisense RNA molecules produced in isolated nuclei from transfected cells, target DNA (immobilized on nitrocellulose filters in triplicate dots, 2 μg per dot) consisted of single-stranded synthetic oligonucleotides rather than TS cDNA. Strand-specific oligonucleotide probes were also used to assess levels of human metallothionein-2 (MT-2) mRNA and antisense RNA as positive and negative controls, respectively. Single-stranded oligonucleotides were immobilized on nitrocellulose filters by dissolving in 6XSSC (16 μg per ml) and applying 125 μl per dot using a BioRad dot-blot apparatus. Unlabeled complementary cDNA probes for GAPDH mRNA (Denhardt *et al.,* 1988) and 18S ribosomal RNA (Behrend *et al,* 1994) were denatured and immobilized on the same nitrocellulose filters (5 μg per dot, triplicate dots) using a previously-described protocol (Koropatnick, 1988). Hybridization of radiolabeled RNA to these dots assessed transcription of GAPDH and 18S rRNA genes, and acted as internal standards against which to measure changes in TS gene transcription. For cells transfected with single-stranded oligonucleotides, TS gene transcription was assessed by hybridization of radiolabeled TS RNA transcripts to immobilized target DNA consisting of a 1.9 kb *Xho 1* fragment isolated from pCHTS-1.

Nitrocellulose filters containing triplicate dots of oligonucleotide and cDNA probes to assess run-on transcription of antisense TS RNA expression vectors, and endogenous TS, MT-2,GAPDH and 18S rRNA genes, were prehybridized in Church buffer for 20 min at 65° C in a Hybaid hybridization chamber. The prehybridization buffer was then removed, 2 mls of radiolabeled RNA resulting from 30 min of run-on transcription in isolated nuclei (in Church hybridization buffer, 4 X $10^6$ cpm per ml) was added, and the filters were hybridized for 48 h at 65° C. The filters were then washed twice at 65°C in posthybridization buffer (40 mM $Na_2HPO_4$, 1% SDS; 20 min per wash). Posthybridization buffer was removed and 8 ml of RNase A (1 μg per ml in 6XSSC) was added and incubated for 30 min at 37° C to reduce signal from unhybridized radiolabeled RNA. After a final wash in posthybridization buffer (10 min, 37° C) filters were blotted dry and bound radioactivity visualized and quantitated using a phosphorimager™ and the ImageQuant™ program (Molecular Dynamics, Sunnyvale, CA). Relative transcription of antisense TS expression vectors, endogenous TS genes, and MT-2 genes was defined as:

$$\text{Relative transcription rate} = \frac{\text{(hybridisation signal from gene of interest)}}{\text{(hybridisation signal for GAPDH or 18S rRNA genes)}}$$

*TS oligonucleotide probes:*

Bases in bold-face (below) form part of restriction endonuclease sites, and are not sense or antisense TS sequences. Numbering indicates the distance from the beginning of the transcription start site.

***TS cDNA nucleotides 111 to 140***

| | | |
|---|---|---|
| *sense TS (JK-5):* | CTAGATGTGGCCGGCTCGGAGCTGCCGCGCCGGCCA | (SEQ ID NO: 11) |
| *antisense TS (JK-2):* | AGCTTGGCCGGCGCGGCAGCTCCGAGCCGGCCACAT | (SEQ ID NO:12) |

***TS cDNA nucleotides 296 to 325***

| | | |
|---|---|---|
| *sense TS (JK-3):* | GTAGAGCTACAGCCTGAGAGATGAATTCCCTCTGCA | (SEQ ID NO:13) |
| *antisense TS* ***(JK-4)***: | AGCTTGCAGAGGGAATTCATCTCTCAGGCTGTAGGT | (SEQ ID NO:14) |

<u>MT-2 oligonucleotide probes (Karin and Richards, 1982):</u>

Sense and antisense oligonuctеotide sequences did not have non-complementary sequences added to the 5' and 3' ends. Numbering indicates the distance from the translation start site.

***MT cDNA nucleotides -14 to 6***

*sense MT:* CTCTTCAGCACGCCATGGAT (SEQ ID NO: 15)

***MT cDNA nucleotides 204 to 223***

*antisense MT:* AGGGTCTACCMCTTGCGC (SEQ ID NO: 16)

**Example 1.2: Antisense oligodeoxynucleotide targeting regions at or near the translation stop site at the 3' end of the TS gene as a method to inhibit growth of human tumour cells**

a) A 20-mer antisense oligodeoxynucleotide (oligo 86) targeted to the translation stop site at the 3' end of the thymidylate synthase mRNA is growth inhibitory (cytostatic) in a human breast cancer cell line (MCF-7 cells). Antisense oligonucleotides of the same length (oligos 90 and 92), targeted to regions at or near the translation start site at the 5' end of the TS mRNA, are not cytostatic (**Figure 1**).

b) A 20-mer antisense oligodeoxynucleotides targeted to the TS mRNA translation start site (oligos 91 and 93) did not inhibit growth of a human cervical carcinoma (HeLa) cell line. In fact, growth was significantly enhanced. Antisense oligodeoxynucleotides targeted to the 3' end of the TS mRNA, including the translation stop site (oligo 86) or a sequence in the 3' untranslated region (oligo 83) significantly inhibited HeLa cell growth. An antisense TS oligonucleotide targeted to another sequence in the 3' untranslated region of TS mRNA (oligo 81) had no effect on HeLa cell growth (**Figures 2 and 3**).

Therefore, no antisense TS oligodeoxynucleotides targeted to the translation start site were successful in inhibiting growth of two different human tumour cell lines (human breast carcinoma MCF-7 cells or human cervical carcinoma HeLa cells). **Two separate antisense TS oligodeoxynucleotides targeted to the 3' end of the TS gene were potent inhibitors of human tumour cell growth.**

**Example 1.3:**

(a) Antisense oligodeoxynucleotide targeting of the thymidylate synthase translation stop site as a method to enhance human tumour cell sensitivity to the toxic effects of Tomudex™(ZD 1694).

Antisense TS oligodeoxynucleotides (oligos 86 and 83) targeting sequences in the 3' untranslated region of TS mRNA enhanced human cervical carcinoma cell sensitivity to Tomudex™. The enhancement in sensitivity was in addition to the directly cytostatic effects of oligos 86 and 83 (Figures 4 and 5).

(b) Antisense oligodeoxynucleotide targeting of the thymidylate synthase translation start site as a method to enhance human cell resistance to the toxic effects of Tomudex™(ZD 1694).

An antisense TS oligodeoxynucleotide (oligo 91) targeting the translation start site of TS mRNA enhanced human cervical carcinoma cell resistance to the toxic effects of Tomudex™ **(Figure 4).**

**Example 1.4: Induction of transcription of genes targeted with antisense nucleic acids as a screening method to identify appropriate target sequences for antisense nucleic acids.**

Human tumour cells appear to compensate for antisense inactivation of specific mRNA by increasing transcription of genes producing the target sequences, a process that can be termed "compensatory transcription", resulting in resistance to the effectiveness of antisense nucleic acids. It has been observed, that TS gene transcription is induced in human MCF-7 breast carcinoma cells by treatment with antisense TS RNA and oligodeoxynucleotides targeted to regions at or near the TS mRNA translation start site. The same phenomenon has been observed in human HeLa cells transiently-transfected with antisense TS oligo 91 (targeted to the translation start site), but not in response to oligo 86 (targeted to the translation stop site) (**Figure 6**). Increased specific gene transcription in response to transfected antisense nucleic acids would indicate that the target sequence is inappropriate to achieve downregulated gene expression. On the other hand, it may be an appropriate sequence to target to achieve upregulated gene expression (to increase resistance to chemotherapeutic drugs, for example, in normal tissues.

In summary, the present invention has demonstrated that antisense oligonucleotides, targeted against selected regions of thymidylate synthase mRNA, can effectively inhibit growth when administered alone. They can also enhance cell killing by Tomudex™. Conversely, antisense oligonucleotides targeted to certain mRNA regions (for example, the translation start site) may either be ineffective, or enhance growth and survival during exposure to Tomudex™. Ineffectiveness may be due to oligonucleotide-induced TS gene transcription. It is essential to identify TS mRNA regions that may be effectively targeted with antisense sequences to inhibit tumour cell growth and enhance the toxicity of anticancer drugs. Furthermore, the mechanism by which antisense sequences targeted to 5' TS mRNA regions induce TS gene transcription has important implications for choosing antisense targets in TS mRNA in particular, and for optimising antisense strategies in general.

**Example 2**

**Example 2.1: Experimental Methods**

*Oligonucleotides*:

Fully phosphorothioated 20-base oligonucleotides were synthesised by ISIS Pharmaceuticals (Carlsbad, California, USA). The 6 nucleotides on either end of the oligomer were methoxyethoxylated in the 2'-position, enhancing hybridisation as well as resistance to exonucleation. The middle 8 nucleotides were not methoxyethoxylated to allow RNase H endonucleation and degradation of mRNA hybridised to the oligomer. ODN 83 is complementary to TS mRNA, starting from a position 136 bases downstream of the translational stop site (5'-GCCAGTGGCAACATCCTTAA-3'). ODN 32 is a randomised sequence of ODN 83 (5'-ATGCGCCAACGGTTCCTAAA-3'), with the same base constituents in random order. A search of available mRNA sequences using the NCBI BLAST search tool revealed that ODN 83 had sequences of 10 or more complementary bases to only human TS mRNA, while ODN 32 had sequences of 10 or more complementary bases to no known mRNAs.

*Radioisotope:*

[6-$^3$H]5-FUdR (specific activity 18.6 Ci/mmol) was purchased from Moravek Biochemicals (Brea, California, USA). This isotope was 99.98% pure upon initial production, with a degradation rate of 0.5-1% per month at -20°C, and was used within 3 months of manufacture.

*Other supplies:*

Cell culture chemicals and nutrients were obtained from Canadian Life Technologies (GIBCO) (Burlington, Ontario, Canada). All other chemicals were obtained from commercial sources. Plasticware was purchased from VWR Canlab (Mississauga, Ontario, Canada) and Fisher Scientific Uniondale, Ontario, Canada).

*Cell Culture:*

Human cervical carcinoma HeLa cells were maintained in D-MEM plus 10% foetal bovine serum and penicillin (50 units/ml)/streptomycin (50 μg/ml). Cultures were incubated in a humidified atmosphere of 5% $CO_2$ at 37°C. Rapidly proliferating cells were utilised for establishing cultures of experimental cells, which were allowed to plate overnight prior to manipulation.

Transfection was performed using lipofectamine (LFA, GIBCO-BRL), a polycationic liposome formulation. Cells to be used for proliferation experiments were plated at a starting cell number of between 0.6 and 1 x $10^5$ cells per 25-cm tissue culture flask, and LFA was used at 3 $\mu$g/ml. For cells in 75-cm flasks, which were to be harvested and extracted for assay of mRNA or TS content, the starting cell number was approximately 8 - 10 x $10^5$, and the LFA concentration was 4 $\mu$g/ml. Prior to transfection, adherent HeLa cells were washed once with PBS and then treated with antisense or scrambled control ODN (50 nM) in the appropriate concentration of LFA in serum-free D-MEM, at 37°C for 6.0h. The cells were then washed once with PBS and cultured in the presence of D-MEM plus 10% FBS. In cells treated with cytotoxic agents, exposure was initiated 24 hours after the removal of LFA/ODN, by addition of 0.2-volume of growth medium containing the agent at 6 times the final concentration. At the time of addition of drug, and after 4 days of incubation, cell numbers were determined from replicate flasks by enumerating with a particle counter (Coulter Electronics, Hialeah, Florida, USA). The proliferation of drug treated cells (fold-increase in cell number) was calculated as a percentage of that of the control cells. $IC_{50}$ and $IC_{90}$ values were determined by interpolation of plotted data.

*RT-PCR to measure TS mRNA:*

RNA was isolated from transfected cells using Trizol™ (GIBCO-BRL). Complementary DNA was synthesised from 1 $\mu$g of total RNA using 200 U of Moloney Murine Leukemia Virus reverse transcriptase (GIBCO BRL) in 50 mM Tris-HCl (pH 8.3), 75 mM KCl, 3 mM $MgCl_2$, 1 mM mixed dNTPs, 100 pmol random primers and 10 mM dithiothreitol at 37°C for 1 hr. The enzyme was inactivated at 95°C for 5 min. The resulting cDNAs (in a volume of 2.5 $\mu$l) were amplified in a polymerase chain reaction (PCR) using 1.25 U of *Taq* DNA polymerase in 50 $\mu$l of 20 mM Tris-HCl pH 8.4), 50 mM KCl, 0.2 mM mixed dNTPs, 2 mM $MgCl_2$, and 50 pmol of primers specific for TS and GAPDH cDNAs. TS and GAPDH cDNAs were amplified together in the same reaction tube to allow the level of housekeeping GAPDH cDNA to be used to determine the relative level of TS mRNA. Twenty-four to 27 cycles of PCR amplification (94°C 45 s, 55°C 30 s, 72°C 90 s) produced fragments of 208 bp and 752 bp using primer sets for TS (forward 5'CACACTTTGGGAGATGCACA3' (SEQ ID NO: 17); reverse 5'CTTTGAAAGCACCCTAAACAGCCAT3'(SEQ ID NO: 18)) and GAPDH (forward 5'TATTGGGCGCCTGGTCACCA3' (SEQ ID NO; 19); reverse 5'CCACCTTCTTGATGTCATCA3') (SEQ ID NO: 20), respectively. PCR products were separated on a 1.2% agarose gel, and transferred to Hybond nylon membrane (Amersham, Canada, Ltd., Oakville, Ontario, Canada) by Southern blotting. Blots were hybridised to [$\alpha$-$^{32}$P]dCTP random primer-labeled probe (pcHTS-1, a generous gift from Dr. K. Takeishi, University of Shizuoka, Shizuoka, Japan; or a cDNA insert recognising glyceraldehyde-3-phosphate dehydrogenase [GAPDH]). Hybridisation signals were, quantified using a Phosphorimager™ and ImageQuant™ (Molecular Dynamics, Sunnyvale, California, USA).

*TS binding assay:*

Cellular content of TS was assayed by binding of [6-$^3$H]5-FdUMP. This method was demonstrated to label total TS unless the cells were pretreated with 5-FU or 5-FUdR. The assay was performed using cells that were treated with antisense ODN 83 or the scrambled control ODN 32. Briefly, cells were harvested by scraping into PBS and resuspending the subsequent pellet in 100 mM $KH_2PO_4$ (pH 7.4); Cells were disrupted by freezing and thawing, followed by sonication. The total protein concentration was determined using Coomassie staining (BioRad reagent) (MI) in order to express results as pmol 5-FdUMP bound per mg total protein. 5-FdUMP binding was assessed in paired lysates from cells transfected with ODN 83 or ODN 32, in separate incubation reactions carried out on different days; however, pairs were always assessed together under the same reaction conditions. On each occasion, the incubation vessel contained 50 $\mu$g of total protein, 75 $\mu$M methylene-$FH_4$, 100 mM mercaptoethanol, 50 mM $KH_2P0_4$ (pH 7.4), and 15 nM [6-$^3$H]5-FdUMP in a final volume of 200 $\mu$l. After 30 min at 37°C, the incubation was stopped by addition of 5 volumes of albumin-coated, activated charcoal. After 10 min (room temperature), this slurry was centrifuged (3000 x g, 30 min, 22°C), and the supernatant re-centrifuged to completely remove particulate matter. Two aliquots of 300 $\mu$l each were removed from the final, clarified supernatant for scintillation counting.

*Statistical analysis:*

Data for cell growth after treatment with ODNs alone, or in combination with cytotoxic drugs, are presented as the mean +/- standard error or standard deviation as determined by Student *t*-test. For determinations of FdUMP binding, differences between paired samples from cells transfected with different ODNs were assessed using a paired *t*-test. This controlled for differences in experimental conditions on each of the 5 occasions that FdUMP binding was assessed. In all cases, significance was chosen *a priori* to be indicated by differences at a confidence level of $p<0.02$.

## References

Almendral, J.M., *et al.* Mol. Cell. Biol. 8:2140-2148, 1988.
Ayusawa, D., *et al.* J. Mol. Biol. 190:559-567, 1996.
Behrend, E.I., *et al.* Cancer Res. 54:832-837,1994.
Calvert, A.H., *et al.* J. Clin. Oncol. 4:1245-1252, 1986.
Chu, E., *et al.* Proc. Natl. Acad. Sci. USA 88:8977-8981, 1991.
Chu, E., *et al.* Mol. Pharm. 43:527-533,1993a.
Chu, E., *et al.* Mol. Cell. Biol. 15:179-185, 1995.
Chu, E., *et* al. Mol. Cell. Biol. 14:207-213, 1994.
Chu, E., *et al.* Proc. Natl. Acad. Sci. USA 90:517-521, 1993b.
Chu, E., *et al.* Cancer Res. 50:5834-5840, 1990.
Church and Gilbert, Proc. Natl. Acad. Sci. USA 81:191-1995, 1984.
Danenberg, P.V., Biochim. Biophys. Acta 473:73-92, 1977.
DeGregori, J., *et al.* Mol. Cell. Biol. 15:4215-4224,1995.
Denhardt, D.T., *et al.* Oncogene 2:55-59, 1988.
Farnham, P.J., *et al.* Biochim. Biophys. Acta Rev. Cancer 1155:125-131,1993.
Hardy, L.W., *et al.* Science 235:448-455, 1987.
Heidelberger, C., *et al.* Adv. Enzymol. 54:58-119, 1983.
Jackman, A.L., *et al.* Adv. Enzyme Regul. 31:13-27, 1991a
Jackman, A.L., *et al.* Cancer Res. 51:5579-5536, 1991b.
Jelinek, W.R., *et al.* Proc. Natl. Acad. Sci. USA 77:1398-1402, 1980.
Jenh, C.-H., *et al.* Mol. Cell. Biol. 5:527-2532, 1985.
Johnson, L.F., J. Cell. Biochem. 54:387-392, 1994.
Karin, M., *et al.* Nucleic Acids Res. 10:3165-3173, 1982.
Keyomarsi, K., *et al. J.* Biol. Chem. 268:15142-15149, 1993.
Kikuchi, K., *et al. J.* Biol. Chem. 267:21505-21511, 1992.
Koropatnick, J., *et al.* Proc. Soc. Exp. Biol. Med. 188:287-300,1988.
Koropatnick, J., *et al.* BioTechniques 22:64-66, 1997.
Koropatnick, J., *et al.* Mol. Biol. Med. 5:69-83, 1988.
Locksin, A., *et al.* Proc. Natl. Acad. Sci. USA 76:750-754.1979.
Maley and Maley, J. Biol. Chem. 235:2968-2970,1960
Marzluff and Huang, IRL Press, Oxford, 89-129, 1984.
M Kay, R.A., *et. al.* Nucleic Acids. Res. 24:411-417,1996.
Mudrak, I., *et al.* Mol. Cell. Biol. 14:1886-1892, 1994.
Navalgund, L.G., *et al.* J. Biol. Chem. 255:7386-7390,1980.
Peters, G.J., *et al.* Cancer Res. 46.20-28, 1986.
Peters, G.J., *et al.*Eur. J. Cancer 30A:1408-1411, 1994.
Rapaport, E., *et al.* Proc. Natl Acad. Sci. USA 89:8577-3580, 1992.
Sambrook, J., *et al.* T. Molecular Cloning: A Laboratory Mannal. Cold Spring Harbor, NY: Cold Spring Harbor Laboratory, 1989.
Suzuki, M., *et al.* Oncology 51:334-338,1994.
Takeishi, K., *et al.* Nucleic Acids Res. 13:2035-2043, 1985.
Voeller, D.M., *et al.* Nucleic Acids Res. 23:869-875,1995.
Volm et al. Anticancer Research 14(3B):1271-1275, 1994.
Volm and Mattern, Anticancer Res. 12:2293-2296, 1992.


## Claims

1. An antisense deoxyoligonucleotide consisting of 8 to 50 nucleotides and comprising a nucleotide sequence complementary to a sequence at or near the translational stop site at the 3' end of a human thymidylate synthase gene, wherein said deoxyoligonucleotide inhibits thymidylate synthase production in a mammalian cell and is nuclease resistant.

2. The antisense deoxyoligonucleotide according to claim 1 consisting of 12 to 40 nucleotides.

3. The antisense deoxyoligonucleotide according to claim 1 consisting of 16 to 30 nucleotides.

4. The antisense deoxyoligonucleotide according to any one of claims 1 to 3, comprising one of the following nucleotide sequences : GCCAGTGGCAACATCCTTAA or AAGCACCCTAAACAGCCATT.

5. The antisense deoxyoligonucleotide according to claim 4 comprising the following nucleotide sequence: GCCAGTGGCAACATCCTTAA

6. The antisense deoxyoligonucleotide according to any one of claims 1 to 5, wherein said deoxyoligonucleotide is phosphorothioated.

7. The antisense deoxyoligonucleotide according to any one of claims 1 to 6, wherein the 6 terminal nucleotides at the 5' and 3' ends of said deoxyoligonucleotide are 2'-methoxyethoxylated.

8. The antisense deoxyoligonucleotide according to any one of claims 1 to 7, wherein said nucleotide sequence is complementary to a region of the thymidylate synthase gene between nucleotide bases 800 and 1536 of the sequence of Figure 7a.

9. The antisense deoxyoligonucleotide according to any one of claims 1 to 8,
wherein said nucleotide sequence is complementary to a region of the thymidylate synthase gene between nucleotide bases 1000 and 1530 of the sequence of Figure 7a.

**10.** The antisense deoxyoligonucleotide according to any one of claims 1 to 9, wherein said nucleotide sequence is complementary to a region of the thymidylate synthase gene between nucleotide bases 1030 and 1460 of the sequence of Figure 7a.

**11.** The antisense deoxyoligonucleotide according to any one of claims 1 to 10, wherein said mammalian cell is a cervical cancer cell.

**12.** The antisense deoxyoligonucleotide according to any one of claims 1 to 10, wherein said mammalian cell is a breast cancer cell.

**13.** The antisense deoxyoligonucleotide according to any one of claims 1 to 12, wherein said mammalian cell is a human cell.

**14.** A pharmaceutical composition comprising the antisense deoxyoligonucleotide according to any one of claims 1 to 13, and a pharmaceutically acceptable diluent or carrier.

**15.** A combination product comprising the antisense deoxyoligonucleotide according to any one of claims 1 to 13, in combination with an anticancer agent.

**16.** The combination product according to claim 15, wherein the anticancer agent is a thymidylate synthase inhibitor, a cytostatic agent or an antiproliferative drug.

**17.** The combination product according to claim 15 or 16, wherein the anticancer agent is a thymidylate synthase inhibitor.

**18.** The combination product according to claim 15 or 16, wherein the anticancer agent is a cytostatic agent.

**19.** The combination product according to claim 15 or 16, wherein the anticancer agent is an antiproliferative drug.

**20.** The combination product according to any one of claims 15 to 17, wherein the anticancer agent is N-(5-[N-(3,4-dihydro-2-methyl-4-oxoquinazolin-6-ylmethyl)-N-methylamino]-2-thenoyl)-L-glutamic acid).

**21.** The combination product according to any one of claims 15 to 17, wherein the anticancer agent is (S)-2-(2-fluoro-4-[N-(4-hydroxy-2,7-dimethylquinazolin-6-ylmethyl)-N-(prop-2-ynyl)amino]benzamido)-4-(1H-1,2,3,4-tetrazol-5-yl) butyric acid.

**22.** The combination product according to any one of claims 15, 16 or 19, wherein the anticancer agent is methotrexate.

**23.** The combination product according to any one of claims 15,16 or 19, wherein the anticancer agent is a fluoropyrimidine.

**24.** The combination product according to claim 23 wherein said fluoropyrimidine is selected from 5-fluorouracil, FUdR, ftorafur and FdUR.

**25.** The combination product according to claim 24, wherein said fluoropyrimidine is 5-fluorouracil.

**26.** The combination product according to claim 24, wherein said fluoropyrimidine is 5-FUdR.

**27.** A pharmaceutical composition comprising the combination product according to any one of claims 15 to 26, and a pharmaceutically acceptable diluent or carrier.

**28.** The antisense deoxyoligonucleotide according to any one of claims 1 to 13 for use in the treatment of cancer in a mammal.

**29.** The antisense deoxyoligonucleotide according to any one of claims 1 to 13, for use to inhibit cell proliferation in a mammal.

**30.** The antisense deoxyoligonucleotide according to claim 29, wherein said cell proliferation is tumour cell proliferation.

31. The antisense deoxyoligonucleotide according to any one of claims 1 to 13 for use to enhance the sensitivity of a mammalian cell to an anticancer agent.

32. The antisense deoxyoligonucleotide according to claim 31, wherein said mammalian cell is a tumour cell.

33. The antisense deoxyoligonucleotide according to claim 31 or 32, wherein said anticancer agent is a fluoropyrimidine.

34. The antisense deoxyoligonucleotide according to claim 33, wherein said fluoropyrimidine is selected from 5-fluorouracil, FudR, ftorafur and FdUR.

35. The antisense deoxyoligonucleotide according to any one of claims 28 to 34, wherein said mammal is a human.

36. The combination product according to any one of claims 15 to 26, for use in the treatment of cancer in a mammal.

37. The combination product according to any one of claims 15 to 26, for use to inhibit cell proliferation in a mammal.

38. The combination product according to claim 37, wherein said cell proliferation is tumour cell proliferation.

39. The combination product according to any one of claims 15 to 26 for use to enhance the sensitivity of a mammalian cell to the anticancer agent of said combination product.

40. The combination product according to any one of claims 15, 16 or 19 for use to enhance the sensitivity of a mammalian cell to said anticancer agent, wherein said anticancer agent is a fluoropyrimidine.

41. The combination product according to claim 40, wherein said fluoropyrimidine is selected from 5-fluorouracil, FudR, ftorafur and FdUR.

42. The combination product according to any one of claims 39 to 41, wherein said mammalian cell is a tumour cell.

43. The combination product according to any one of claims 36 to 42, wherein the mammal is a human.

44. Use of the antisense deoxyoligonucleotide according to any one of claims 1 to 13, for the preparation of a medicament.

45. The use according to claim 44, wherein the medicament is for the treatment of cancer in a mammal.

46. The use according to claim 44, wherein the medicament is for the inhibition of cell proliferation in a mammal.

47. The use according to claim 46, wherein said cell proliferation is tumour cell proliferation.

48. The use according to claim 44, wherein said medicament is for enhancing the sensitivity of a mammalian cell to an anticancer agent.

49. The use according to claim 48, wherein said mammalian cell is a tumour cell.

50. The use according to claim 48 or 49, wherein said anticancer agent is a fluoropyrimidine.

51. The use according to claim 50, wherein said fluoropyrimidine is selected from 5-fluorouracil, FudR, ftorafur and FdUR.

52. The use according to any one of claims 45 to 51, wherein the mammal is a human.

53. Use of the combination product according to any one of claims 15 to 26, in the preparation of a medicament.

54. The use according to claim 53, wherein the medicament is for the treatment of cancer in a mammal.

55. The use according to claim 53, wherein the medicament is for the inhibition of cell proliferation in a mammal.

56. The use according to claim 55, wherein said cell proliferation is tumour cell proliferation.

**57.** The use according to claim 53, wherein said medicament is for enhancing the sensitivity of a mammalian cell to said anticancer agent of the combination product.

**58.** The use of a combination product according to any one of claims 15,16, or 19 to prepare a medicament for enhancing the sensitivity of a mammalian cell to said anticancer agent of the combination product, wherein said anticancer agent is a fluoropyrimidine.

**59.** The use according to claim 58, wherein said fluropyrimidine is selected from 5-fluorouracil, FudR, ftorafur and FdUR.

**60.** The use according to any one of claims 57 to 59, wherein said mammalian cell is a tumour cell.

**61.** The use according to any one of claims 54 to 60, wherein the mammal is a human.

**Patentansprüche**

**1.** Ein Antisense-Deoxyoligonukleotid bestehend aus 8 bis 50 Nukleotiden, das eine Nukleotidsequenz umfasst, welche komplementär zu einer Sequenz an oder nahe der Translationsendstelle am 3'-Ende eines menschlichen Thymidylatsynthasegens ist, **gekennzeichnet dadurch, dass** das besagte Deoxyoligomikleotid die Thymidylatsynthaseproduktion in einer Säugetierzelle hemmt und nukleaseresistent ist.

**2.** Antisense-Deoxyoligonukleotid nach Anspruch 1, bestehend aus 12 bis 40 Nukleotiden.

**3.** Antisense-Deoxyoligonukleotid nach Anspruch 1, bestehend aus 16 bis 30 Nukleotiden.

**4.** Antisense-Deoxyoligonukleotid nach einem der Ansprüche 1 bis 3, das [handschriftliche Einfügung:] *eine der folgenden* Nukleotidsequenz*en* umfasst: GCCAGTGGCAACATCCTTAA *oder* AAGCACCCTAAACAGCC.

**5.** Antisense-Deoxyoligonukleotid nach Anspruch 4, das die [handschriftliche Einfügung:] *folgende* Nukleotidsequenz umfasst: GCCAGTGGCAACATCCTTAA.

**6.** Antisense-Deoxyoligonukleotid nach einem der Ansprüche 1 bis 5, wobei das besagte Deoxyoligonukleotid Phosphorothiat-modifiziert ist.

**7.** Antisense-Deoxyoligonukleotid nach einem der Ansprüche 1 bis 6, **gekennzeichnet dadurch, dass** die 6 Endnukleotide an den 5'- und 3'-Enden des besagten Deoxyoligonukleotids 2'-methoxyethoxyliert sind.

**8.** Antisense-Deoxyoligonukleotid nach einem der Ansprüche 1 bis 7, **gekennzeichnet dadurch, dass** die besagte Nukleotidsequenz komplementär ist zu einem Bereich des Thymidylatsynthasegens zwischen den Nukleotidbasen 800 und 1536 der Sequenz in Abbildung 7a.

**9.** Antisense-Deoxyoligonukleotid nach einem der Ansprüche 1 bis 8, **gekennzeichnet dadurch, dass** die besagte Nukleotidsequenz komplementär ist zu einem Bereich des Thymidylatsynthasegens zwischen den Nukleotidbasen 1000 und 1530 der Sequenz in Abbildung 7a.

**10.** Antisense-Deoxyoligonukleotid nach einem der Ansprüche 1 bis 9, **gekennzeichnet dadurch, dass** die besagte Nukleotidsequenz komplementär ist zu einem Bereich des Thymidylatsynthasegens zwischen den Nukleotidbasen 1030 und 1460 der Sequenz in Abbildung 7a.

**11.** Antisense-Deoxyoligonukleotid nach einem der Ansprüche 1 bis 10, **gekennzeichnet dadurch, dass** die besagte Säugetierzelle eine Gebärmutterhalskrebszelle ist.

**12.** Antisense-Deoxyoligonukleotid nach einem der Ansprüche 1 bis 10, **gekennzeichnet dadurch, dass** die besagte Säugetierzelle eine Brustkrebszelle ist.

**13.** Antisense-Deoxyoligonukleotid nach einem der Ansprüche 1 bis 12, **gekennzeichnet dadurch, dass** die besagte Säugetierzelle eine menschliche Zelle ist.

**14.** Eine pharmazeutische Rezeptur bestehend aus dem Antisense-Deoxyoligonukleotid nach einem der Ansprüche 1 bis 13 und einem pharmazeutisch zulässigen Verdünnungsmittel oder Träger.

**15.** Ein Kombinationsprodukt bestehend aus dem Antisense-Deoxyoligonukleotid nach einem der Ansprüche 1 bis 13 in Kombination mit einem Antikrebswirkstoff.

**16.** Kombinationsprodukt nach Anspruch 15, **gekennzeichnet dadurch, dass** der Antikrebswirkstoff ein Thymidylatsynthasehemmstoff, ein zytostatischer Wirkstoff oder ein antiproliferatives Medikament ist.

**17.** Kombinationsprodukt nach Anspruch 15 oder 16, **gekennzeichnet dadurch, dass** der Antikrebswirkstoff ein Thymidylatsynthasehemmstoff ist.

**18.** Kombinationsprodukt nach Anspruch 15 oder 16, **gekennzeichnet dadurch, dass** der Antikrebswirkstoff ein zytostatischer Wirkstoff ist.

**19.** Kombinationsprodukt nach Anspruch 15 oder 16, **gekennzeichnet dadurch, dass** der Antikrebswirkstoff ein antiproliferatives Medikament ist.

**20.** Kombinationsprodukt nach einem der Ansprüche 15 bis 17, **gekennzeichnet dadurch, dass** der Antikrebswirkstoff eine N-(5-[N-(3,4-dihydro-2-methyl-4-oxoquinazolin-6-ylmethyl)-N-methylammo]-2-thenoyl)-L-Glutaminsäureist.

**21.** Kombinationsprodukt nach einem der Ansprüche 15 bis 17, **gekennzeichnet dadurch, dass** der Antikrebswirkstoff eine (S)-2-(2-fluoro-4-[N-(4-hydroxy-2,7-dimethylquinazolin-6-ylmethyl)-N-(prop-2-ynyl)amino]benzamido)-4-(1H-1,2,3,4-tetrazol-5-yl)Buttersäure ist.

**22.** Kombinationsprodukt nach einem der Ansprüche 15, 16 oder 19, **gekennzeichnet dadurch, dass** der Antikrebswirkstoff Methotrexat ist.

**23.** Kombinationsprodukt nach einem der Ansprüche 15, 16 oder 19, **gekennzeichnet dadurch, dass** der Antikrebswirkstoff Fluoropyrimidin ist.

**24.** Kombinationsprodukt nach Anspruch 23, **gekennzeichnet dadurch, dass** das besagte Fluoropyrimidin aus 5-Fluorouracil, FUdR, Ftorafur und FdUR gewählt wird.

**25.** Kombinationsprodukt nach Anspruch 24, **gekennzeichnet dadurch, dass** das besagte Fluoropyrimidin 5-Fluorouracil ist.

**26.** Kombinationsprodukt nach Anspruch 24, **gekennzeichnet dadurch, dass** das besagte Fluoropyrimidin 5-FUdR ist.

**27.** Eine pharmazeutische Rezeptur bestehend aus dem Kombinationsprodukt nach einem der Ansprüche 15 bis 26 und einem pharmazeutisch zulässigen Verdünnungsmittel oder Träger.

**28.** Antisense-Deoxyoligonukleotid nach einem der Ansprüche 1 bis 13 für den Gebrauch bei der Behandlung von Krebs bei Säugetieren.

**29.** Antisense-Deoxyoligonukleotid nach einem der Ansprüche 1 bis 13 für den Gebrauch bei der Hemmung von Zellvermehrung bei Säugetieren.

**30.** Antisense-Deoxyoligonukleotid nach Anspruch 29, **gekennzeichnet dadurch, dass** es sich bei der besagten Zellvermehrung um die Vermehrung von Tumorzellen handelt.

**31.** Antisense-Deoxyoligonukleotid nach einem der Ansprüche 1 bis 13 für den Gebrauch bei der Erhöhung der Empfindlichkeit einer Säugetierzelle gegen einen Antikrebswirkstoff.

**32.** Antisense-Deoxyoligonukleotid nach Anspruch 31, **gekennzeichnet dadurch, dass** die besagte Säugetierzelle eine Tumorzelle ist.

**33.** Antisense-Deoxyoligonukleotid nach Anspruch 31 oder 32, **gekennzeichnet dadurch, dass** der besagte Antikrebswirkstoff ein Fluoropyrimidin ist.

**34.** Antisense-Deoxyoligonukleotid nach Anspruch 33, **gekennzeichnet dadurch, dass** das besagte Fluoropyrimidin aus 5-Fluorouracil, FudR, Ftorafur und FdUR gewählt wird.

**35.** Antisense-Deoxyoligonukleotid nach einem der Ansprüche 28 bis 34, **gekennzeichnet dadurch, dass** das besagte Säugetier ein Mensch ist.

**36.** Kombinationsprodukt nach einem der Ansprüche 15 bis 26 für den Gebrauch bei der Behandlung von Krebs bei Säugetieren.

**37.** Kombinationsprodukt nach einem der Ansprüche 15 bis 26 für den Gebrauch bei der Hemmung der Zellvermehrung bei Säugetieren.

**38.** Kombinationsprodukt nach Anspruch 37, **gekennzeichnet dadurch, dass** es sich bei der besagten Zellvermehrung um die Vermehrung von Tumorzellen handelt.

**39.** Kombinationsprodukt nach einem der Ansprüche 15 bis 26 für den Gebrauch bei der Erhöhung der Empfindlichkeit einer Säugetierzelle gegen den Antikrebswirkstoff des besagten Kombinationsprodukts.

**40.** Kombinationsprodukt nach einem der Ansprüche 15, 16 oder 19 für den Gebrauch bei der Erhöhung der Empfindlichkeit einer Säugetierzelle gegen den besagten Antikrebswirkstoff, **gekennzeichnet dadurch, dass** der besagte Antikrebswirkstoff ein Fluoropyrimidin ist.

**41.** Kombinationsprodukt nach Anspruch 40, **gekennzeichnet dadurch, dass** das besagte Fluoropyrimidin aus 5-Fluorouracil, FudR, Ftorafur und FdUR gewählt wird.

**42.** Kombinationsprodukt nach einem der Ansprüche 39 bis 41, **gekennzeichnet dadurch, dass** die besagte Säugetierzelle eine Tumorzelle ist.

**43.** Kombinationsprodukt nach einem der Ansprüche 36 bis 42, **gekennzeichnet dadurch, dass** das besagte Säugetier ein Mensch ist.

**44.** Gebrauch des Antisense-Deoxyoligonukleotids nach einem der Ansprüche 1 bis 13 für die Bereitstellung eines Medikaments.

**45.** Gebrauch nach Anspruch 44, **gekennzeichnet dadurch, dass** das Medikament für die Behandlung von Krebs bei Säugetieren gedacht ist.

**46.** Gebrauch nach Anspruch 44, **gekennzeichnet dadurch, dass** das Medikament für die Hemmung der Zellvermehrung bei Säugetieren gedacht ist.

**47.** Gebrauch nach Anspruch 46, **gekennzeichnet dadurch, dass** es sich bei der besagten Zellvermehrung um die Vermehrung von Tumorzellen handelt.

**48.** Gebrauch nach Anspruch 44, **gekennzeichnet dadurch, dass** das besagte Medikament für die Erhöhung der Empfindlichkeit einer Säugetierzelle gegen einen Antikrebswirkstoff gedacht ist.

**49.** Gebrauch nach Anspruch 48, **gekennzeichnet dadurch, dass** die besagte Säugetierzelle eine Tumorzelle ist.

**50.** Gebrauch nach Anspruch 48 oder 49, **gekennzeichnet dadurch, dass** der besagte Antikrebswirkstoff ein Fluoropyrimidin ist.

**51.** Gebrauch nach Anspruch 50, **gekennzeichnet dadurch, dass** das besagte Fluoropyrimidin aus 5-Fluorouracil, FudR, Ftorafur und FdUR gewählt wird.

**52.** Gebrauch nach einem der Ansprüche 45 bis 51, **gekennzeichnet dadurch, dass** das Säugetier ein Mensch ist.

**53.** Gebrauch des Kombinationsprodukts nach einem der Ansprüche 15 bis 26 bei der Bereitstellung eines Medikaments.

**54.** Gebrauch nach Anspruch 53, **gekennzeichnet dadurch, dass** das Medikament für die Behandlung von Krebs bei Säugetieren gedacht ist.

**55.** Gebrauch nach Anspruch 53, **gekennzeichnet dadurch, dass** das Medikament für die Hemmung der Zellvermehrung bei Säugetieren gedacht ist.

**56.** Gebrauch nach Anspruch 55, **gekennzeichnet dadurch, dass** es sich bei der besagten Zellvermehrung um die Vermehrung von Tumorzellen handelt.

**57.** Gebrauch nach Anspruch 53, **gekennzeichnet dadurch, dass** das besagte Medikament für die Erhöhung der Empfindlichkeit einer Säugetierzelle gegen den besagten Antikrebswirkstoff des Kombinationsprodukts gedacht ist.

**58.** Gebrauch des Kombinationsprodukts nach einem der Ansprüche 15, 16 oder 19 für die Bereitstellung eines Medikaments zur Erhöhung der Empfindlichkeit einer Säugetierzelle gegen den besagten Antikrebswirkstoff des Kombinationsprodukts, **gekennzeichnet dadurch, dass** der besagte Antikrebswirkstoff ein Fluoropyrimidin ist.

**59.** Gebrauch nach Anspruch 58, **gekennzeichnet dadurch, dass** das besagte Fluoropyrimidin aus 5-Fluorouracil, FudR, Ftorafur und FdUR gewählt wird.

**60.** Gebrauch nach einem der Ansprüche 57 bis 59, **gekennzeichnet dadurch, dass** die besagte Säugetierzelle eine Tumorzelle ist.

**61.** Gebrauch nach einem der Ansprüche 54 bis 60, **gekennzeichnet dadurch, dass** das Säugetier ein Mensch ist.

## Revendications

**1.** Un désoxyoligonucléotide anti-sens formé de 8 à 50 nucléotides et comprenant une séquence de nucléotides qui complète une séquence située sur le codon-stop de traduction, ou près de celui-ci, à l'extrémité 3' d'un gène de la synthase de l'acide thymidylique, en quoi ledit désoxyoligonucléotide inhibe la production de la synthase de l'acide thymidylique dans une cellule mammalienne et est résistant à la nucléase.

**2.** Le désoxyoligonucléotide anti-sens conformément à la revendication 1, formé de 12 à 40 nucléotides.

**3.** Le désoxyoligonucléotide anti-sens conformément à la revendication 1, formé de 16 à 30 nucléotides.

**4.** Le désoxyoligonucléotide anti-sens conformément à l'une des revendications 1 à 3, formé de l'une des séquences de nucléotides suivantes : GCCAGTGGCAACATCCTTAA ou AAGCACCCTAAACAGCC

**5.** Le désoxyoligonucléotide anti-sens conformément à la revendication 4, formé de la séquence nucléotidique suivante : GCCAGTGGCAACATCCTTAA

**6.** Le désoxyoligonucléotide anti-sens conformément à l'une des revendications 1 à 5, en quoi ledit désoxyoligonucléotide est porteur de phosphorothioate.

**7.** Le désoxyoligonucléotide anti-sens conformément à l'une des revendications 1 à 6, en quoi les 6 nucléotides terminaux aux extrémités 5' et 3' dudit désoxyoligonucléotide sont porteurs de 2'-methoxyethoxylate.

**8.** Le désoxyoligonucléotide anti-sens conformément à l'une des revendications 1 à 7, en quoi ladite séquence nucléotidique est complémentaire à une région du gène de la synthase de l'acide thymidylique entre les bases nucléotidiques 800 et 1536 de la séquence figurant sur le diagramme 7a.

**9.** Le désoxyoligonucléotide anti-sens conformément à l'une des revendications 1 à 8, en quoi ladite séquence nucléotidique est complémentaire à une région du gène de la synthase de l'acide thymidylique entre les bases nucléotidiques 1000 et 1530 de la séquence figurant sur le diagramme 7a.

10. Le désoxyoligonucléotide anti-sens conformément à l'une des revendications 1 à 9, en quoi ladite séquence nucléotidique est complémentaire à une région du gène de la synthase de l'acide thymidylique entre les bases nucléotidiques 1030 et 1460 de la séquence figurant sur le diagramme 7a.

11. Le désoxyoligonucléotide anti-sens conformément à l'une des revendications 1 à 10, en quoi ladite cellule mammalienne est une cellule de cancer du col de l'utérus.

12. Le désoxyoligonucléotide anti-sens conformément à l'une des revendications 1 à 10, en quoi ladite cellule mammalienne est une cellule de cancer du sein.

13. Le désoxyoligonucléotide anti-sens conformément à l'une des revendications 1 à 12, en quoi ladite cellule mammalienne est une cellule humaine.

14. Une composition pharmaceutique qui comprend le désoxyoligonucléotide anti-sens conformément à l'une des réclamations 1 à 13, et. un diluant ou un vecteur acceptable sur le plan pharmaceutique.

15. Un produit composite comprenant le désoxyoligonucléotide anti-sens conformément à l'une des revendications 1 à 13, combiné à un agent anticancéreux.

16. Le produit composite conformément à la revendication 15, en quoi l'agent anticancéreux est un inhibiteur de la synthase de l'acide thymidylique, un cytostatique ou un médicament antiprolifératif.

17. Le produit composite conformément à la revendication 15 ou 16, en quoi l'agent anticancéreux est un inhibiteur de la synthase de l'acide thymidylique.

18. Le produit composite conformément à la revendication 15 ou 16, en quoi l'agent anticancéreux est cytostatique.

19. Le produit composite conformément à la revendication 15 ou 16, en quoi l'agent anticancéreux est un médicament antiprolifératif.

20. Le produit composite conformément à l'une des revendications 15 à 17, en quoi l'agent anticancéreux est l'acide N-(5-[N-(3,4-dihydro-2-méthyl-4-oxoquinazoline-6-ylméthyl)-N-méthylamino]-2-thénoyl)-L-glutamique.

21. Le produit composite conformément à l'une des revendications 15 à 17, en quoi l'agent anticancéreux est l'acide (S)-2-(2-fluoro-4-[N-(4-hydroxy-2,7-diméthylquinazoline-6-ylméthyl)-N-(prop-2-ynyl) amino] benzamido)-4-(1H-1,2,3,4-tétrazol-5-yl)butyrique.

22. Le produit composite conformément à l'une des revendications 15, 16 ou 19, en quoi l'agent anticancéreux est le méthotrexate.

23. Le produit composite conformément à l'une des revendications 15, 16 ou 19, en quoi l'agent anticancéreux est une fluoropyrimidine.

24. Le produit composite conformément à la revendication 23, en quoi ladite fluoropyrimidine est le 5-fluorouracil, le FUdR, le ftorafur ou le FdUR.

25. Le produit composite conformément à la revendication 24, en quoi ladite fluoropyrimidine est le 5-fluorouracil.

26. Le produit composite conformément à la revendication 23, en quoi ladite fluoropyrimidine est le 5-FUdR.

27. Une composition pharmaceutique qui comprend le produit composite conformément à l'une des réclamations 15 à 26, et un diluant ou un vecteur acceptable sur le plan pharmaceutique.

28. Le désoxyoligonucléotide anti-sens conformément à l'une des revendications 1 à 13, utilisé pour le traitement du cancer chez un mammifère.

29. Le désoxyoligonucléotide anti-sens conformément à l'une des revendications 1 à 13, utilisé pour inhiber la prolifération cellulaire chez un mammifère.

**30.** Le désoxyoligonucléotide anti-sens conformément à la revendication 29, en quoi la prolifération cellulaire est la prolifération de cellules tumorales.

**31.** Le désoxyoligonucléotide anti-sens conformément à l'une des revendications 1 à 13, utilisé pour augmenter la réceptivité d'une cellule mammalienne à un agent anticancéreux.

**32.** Le désoxyoligonucléotide anti-sens conformément à la revendication 31, en quoi ladite cellule mammalienne est une cellule tumorale.

**33.** Le désoxyoligonucléotide anti-sens conformément à la revendication 31 ou 32, en quoi ledit agent anticancéreux est une fluoropyrimidine.

**34.** Le désoxyoligonucléotide anti-sens conformément à la revendication 33, en quoi ladite fluoropyrimidine est le 5-fluorouracil, le FudR, le ftorafur ou le FdUR.

**35.** Le désoxyoligonucléotide anti-sens conformément à l'une des revendications 28 à 34, en quoi ledit mammifère est un humain.

**36.** Le produit composite conformément à l'une des revendications 15 à 26, utilisé pour le traitement du cancer chez un mammifère.

**37.** Le produit composite conformément à l'une des revendications 15 à 26, utilisé pour inhiber la prolifération cellulaire chez un mammifère.

**38.** Le produit composite conformément à la revendication 37, en quoi la prolifération cellulaire est la prolifération de cellules tumorales.

**39.** Le produit composite conformément à l'une des revendications 15 à 26, utilisé pour augmenter la réceptivité d'une cellule mammalienne à l'agent anticancéreux dudit produit composite.

**40.** Le produit composite conformément à l'une des revendications 15, 16 ou 19, utilisé-pour augmenter la réceptivité d'une cellule mammalienne audit agent anticancéreux, en quoi ledit agent anticancéreux est une fluoropyrimidine.

**41.** Le produit composite conformément à la revendication 40, en quoi ladite fluoropyrimidine est le 5-fluorouracil, le FudR, le ftorafur ou le FdUR.

**42.** Le produit composite conformément à l'une des revendications 39 à 41, en quoi ladite cellule mammalienne est une cellule tumorale.

**43.** Le produit composite conformément à l'une des revendications 36 à 42, en quoi ledit mammifère est un humain.

**44.** L'utilisation du désoxyoligonucléotide anti-sens conformément à l'une des revendications 1 à 13, pour la préparation d'un médicament.

**45.** L'utilisation conformément à la revendication 44, en quoi le médicament est destiné au traitement du cancer chez un mammifère.

**46.** L'utilisation conformément à la revendication 44, en quoi le médicament est destiné à inhiber la prolifération cellulaire chez un mammifère.

**47.** L'utilisation conformément à la revendication 46, en quoi la prolifération cellulaire est la prolifération de cellules tumorales.

**48.** L'utilisation conformément à la revendication 44, en quoi ledit médicament est destiné à augmenter la réceptivité d'une cellule mammalienne à un agent anticancéreux.

**49.** L'utilisation conformément à la revendication 48, en quoi ladite cellule mammalienne est une cellule tumorale.

**50.** L'utilisation conformément à la revendication 48 ou 49, en quoi ledit agent anticancéreux est une fluoropyrimidine.

**51.** L'utilisation conformément à la revendication 50, en quoi ladite fluoropyrimidine est le 5-fluorouracil, le FudR, le ftorafur ou le FdUR.

**52.** L'utilisation conformément à l'une des revendications 45 à 51, en quoi le mammifère est un humain.

**53.** L'utilisation du produit composite conformément à l'une des revendications 15 à 26 pour la préparation d'un médicament.

**54.** L'utilisation conformément à la revendication 53, en quoi le médicament est destiné au traitement du cancer chez un mammifère.

**55.** L'utilisation conformément à la revendication 53, en quoi le médicament est destiné à inhiber la prolifération cellulaire chez un mammifère.

**56.** L'utilisation conformément à la revendication 55, en quoi la prolifération cellulaire est la prolifération de cellules tumorales.

**57.** L'utilisation conformément à la revendication 53, en quoi ledit médicament est destiné à augmenter la réceptivité d'une cellule mammalienne audit agent anticancéreux du produit composite.

**58.** L'utilisation d'un produit composite conformément à l'une des revendications 15, 16 ou 19, pour la préparation d'un médicament utilisé pour augmenter la réceptivité d'une cellule mammalienne audit agent anticancéreux du produit composite, en quoi ledit agent anticancéreux est une fluoropyrimidine.

**59.** L'utilisation conformément à la revendication 58, en quoi ladite fluoropyrimidine est le 5-fluorouracil, le FudR, le ftorafur ou le FdUR.

**60.** L'utilisation conformément à l'une des revendications 57 à 59, en quoi ladite cellule mammalienne est une cellule tumorale.

**61.** L'utilisation conformément à l'une des revendications 54 à 60, en quoi le mammifère est un humain.

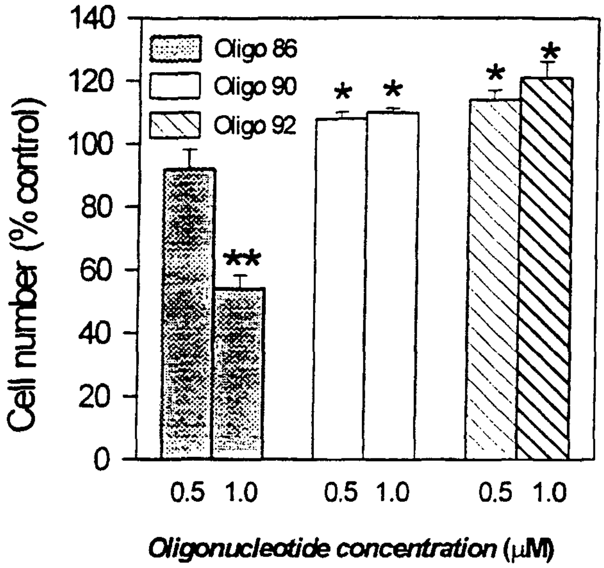
140
120
100
80
60
40
20
0
Cell number (% control)
Oligo 86
Oligo 90
Oligo 92
*
**
0.5 1.0
0.5 1.0
0.5 1.0
Oligonucleotide concentration (μM)

**Figure 1**

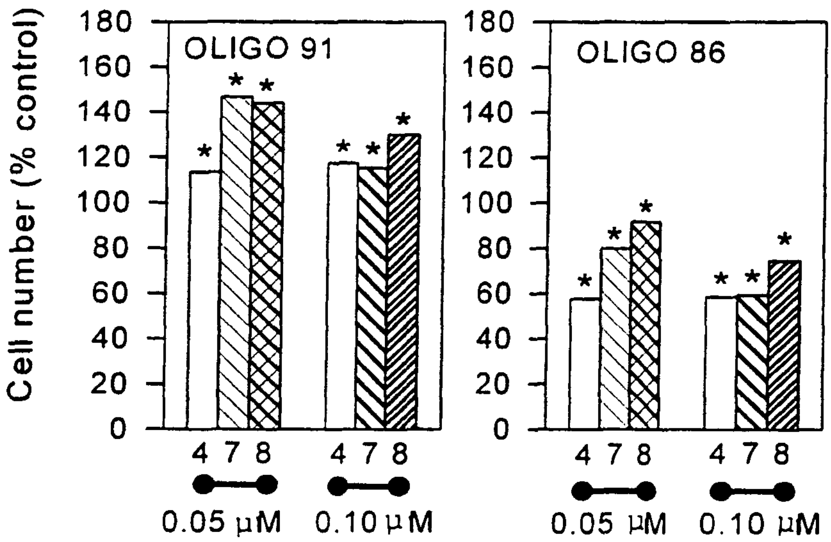
Cell number (% control)
180
160
140
120
100
80
60
40
20
0
OLIGO 91
4 7 8
4 7 8
0.05 µM
0.10 µM
OLIGO 86
4 7 8
4 7 8
0.05 µM
0.10 µM

Figure 2

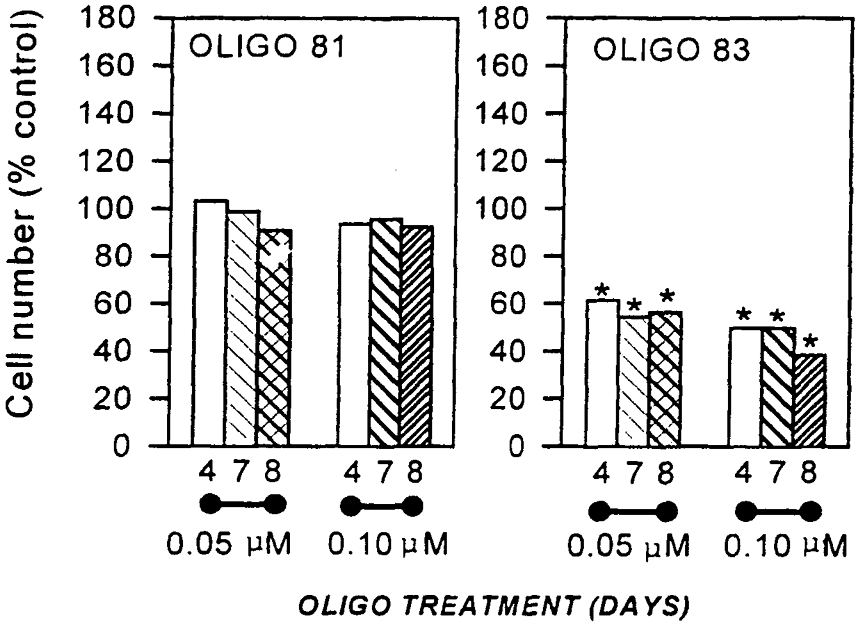
Cell number (% control)
180
160
140
120
100
80
60
40
20
0
OLIGO 81
OLIGO 83
4 7 8
0.05 µM
0.10 µM
OLIGO TREATMENT (DAYS)

**Figure 3**

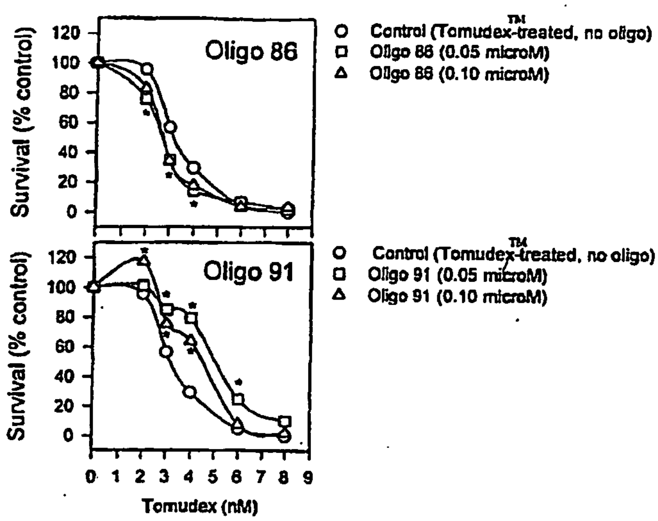
Survival (% control)
120
100
80
60
40
20
0
Oligo 86
Control (Tomudex™-treated, no oligo)
Oligo 86 (0.05 microM)
Oligo 86 (0.10 microM)
Survival (% control)
Oligo 91
Control (Tomudex™-treated, no oligo)
Oligo 91 (0.05 microM)
Oligo 91 (0.10 microM)
0 1 2 3 4 5 6 7 8 9
Tomudex (nM)

**Figure 4**

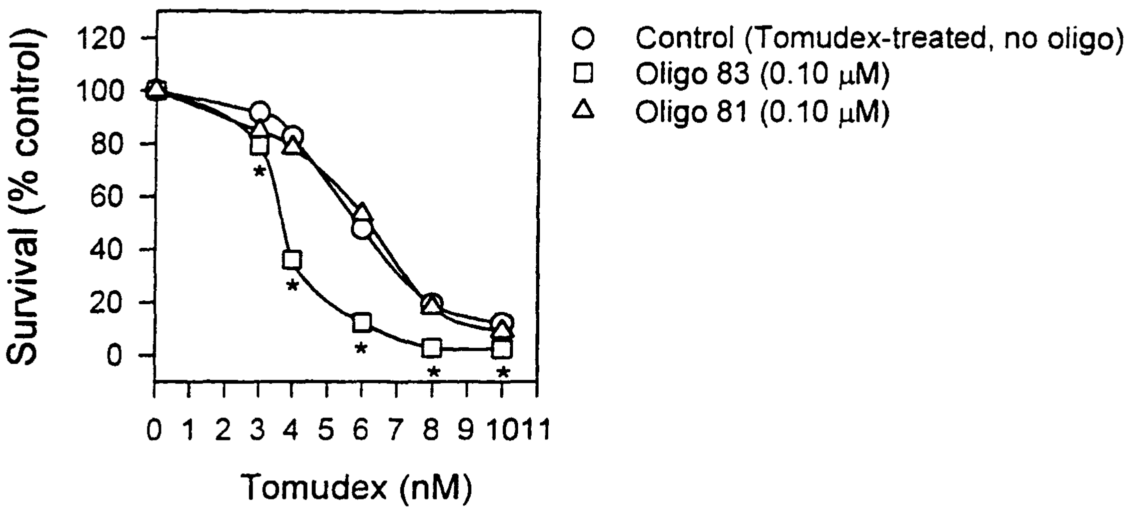
Survival (% control)
120
100
80
60
40
20
0
0 1 2 3 4 5 6 7 8 9 10 11
Tomudex (nM)
Control (Tomudex-treated, no oligo)
Oligo 83 (0.10 μM)
Oligo 81 (0.10 μM)

**Figure 5**

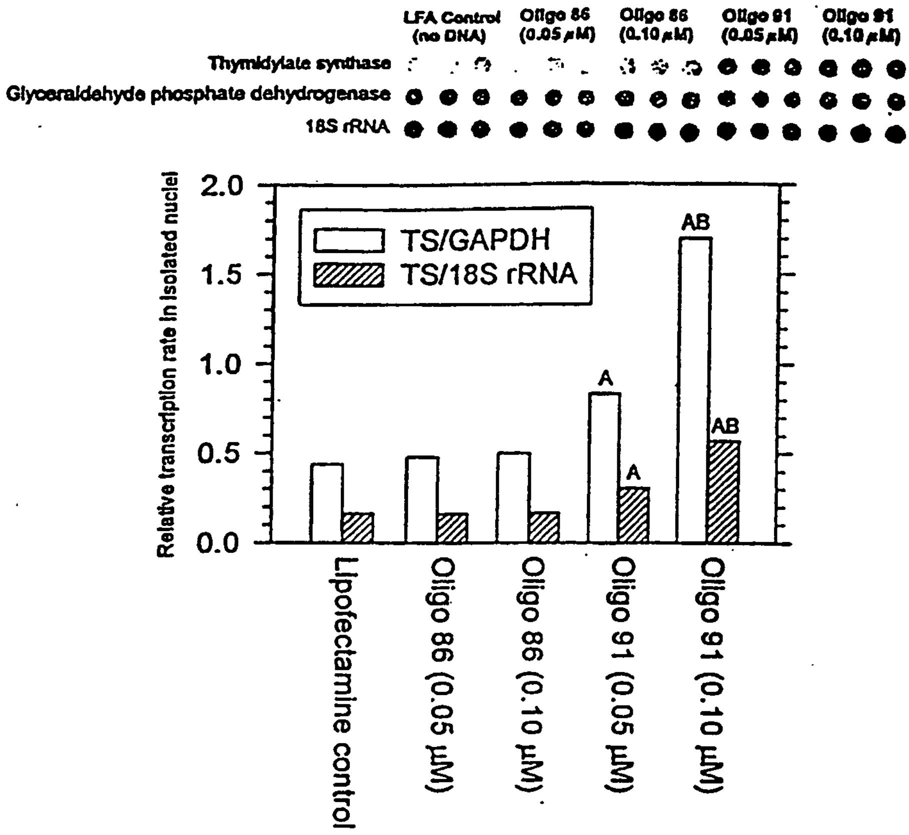
LFA Control (no DNA)
Oligo 86 (0.05 µM)
Oligo 86 (0.10 µM)
Oligo 91 (0.05 µM)
Oligo 91 (0.10 µM)
Thymidylate synthase
Glyceraldehyde phosphate dehydrogenase
18S rRNA
Relative transcription rate in isolated nuclei
2.0
1.5
1.0
0.5
0.0
TS/GAPDH
TS/18S rRNA
A
AB
Lipofectamine control
Oligo 86 (0.05 µM)
Oligo 86 (0.10 µM)
Oligo 91 (0.05 µM)
Oligo 91 (0.10 µM)

Figure 6

**(first 80 bases in TS cDNA/mRNA not shown)**

Oligo 91

Oligo 93 Oligo 90

81 ...gcctccgtcc cccgcccgcc gcgcc**atg**cc tgtggccggc tcggagctgc cgcgccggcc...

Oligo 92

*Translation start*

(thymidylate synthase mRNA sequence, 7 exons)

Oligo 86

1031 ...tggaaatggc tgtt**tag**ggt gctttcaaag gagcttgaag gatattgtca gtctttaggg

*Translation stop*

1091 gttgggctgg atgccgaggt aaaagttctt tttgctctaa aagaaaaagg aactaggtca

Oligo 83

1151 aaaatctgtc cgtgacctat cagttattaa ttttaagga tgttgccact ggcaaatgta

1211 actgtgccag ttctttccat aataaaaggc tttgagttaa ctcactgagg gtatctgaca

1271 atgctgaggt tatgaacaaa gtgaggagaa tgaaatgtat gtgctcttag caaaaacatg

1331 tatgtgcatt tcaatcccac gtacttataa agaaggttgg tgaatttcac aagctatttt

Oligo 81

1391 tggaatattt ttagaatatt ttaagaattt cacaagctat tccctcaaat ctgagggagc tgagtaacac

1461 catcgatcat gatgtagagt gtggttatga actttatagt tgttttatat gttgctataa taaagaagtg ttctgc

**Figure 7**

Entire sequence of human mRNA(cDNA) for thymidylate synthase (EC 2.1.1.45) bases 1 to 1536

```
gggggggggg ggaccacttg gcctgcctcc gtcccgccgc gccacttggc ctgcctccgt 60
cccgccgcgc cacttcgcct gcctccgtcc cccgcccgcc gcgccatgcc tgtggccggc 120
tcggagctgc cgcgccggcc cttgcccccc gccgcacagg agcgggacgc cgagccgcgt 180
ccgccgcacg gggagctgca gtacctgggg cagatccaac acatcctccg ctgcggcgtc 240
aggaaggacg accgcacggg caccggcacc ctgtcggtat tcggcatgca ggcgcgctac 300
agcctgagag atgaattccc tctgctgaca accaaacgtg tgttctggaa gggtgttttg 360
gaggagttgc tgtggtttat caagggatcc acaaatgcta aagagctgtc ttccaaggga 420
gtgaaaatct gggatgccaa tggatcccga gactttttgg acagcctggg attctccacc 480
agagaagaag gggacttggg cccagtttat ggcttccagt ggaggcattt tggggcagaa 540
tacagagata tggaatcaga ttattcagga cagggagttg accaactgca aagagtgatt 600
gacaccatca aaaccaaccc tgacgacaga agaatcatca tgtgcgcttg gaatccaaga 660
gatcttcctc tgatggcgct gcctccatgc catgccctct gccagttcta tgtggtgaac 720
agtgagctgt cctgccagct gtaccagaga tcgggagaca tgggcctcgg tgtgcctttc 780
aacatcgcca gctacgccct gctcacgtac atgattgcgc acatcacggg cctgaagcca 840
ggtgacttta tacacacttt gggagatgca catatttacc tgaatcacat cgagccactg 900
aaaattcagc ttcagcgaga acccagacct ttcccaaagc tcaggattct tcgaaaagtt 960
gagaaaattg atgacttcaa agctgaagac tttcagattg aagggtacaa tccgcatcca 1020
actattaaaa tggaaatggc tgtttagggt gctttcaaag gagcttgaag gatattgtca 1080
gtctttaggg gttgggctgg atgccgaggt aaaagttctt tttgctctaa aagaaaaagg 1140
aactaggtca aaaatctgtc cgtgacctat cagttattaa tttttaagga tgttgccact 1200
ggcaaatgta actgtgccag ttctttccat aataaaaggc tttgagttaa ctcactgagg 1260
gtatctgaca atgctgaggt tatgaacaaa gtgaggagaa tgaaatgtat gtgctcttag 1320
caaaaacatg tatgtgcatt tcaatcccac gtacttataa agaaggttgg tgaatttcac 1380
aagctatttt tggaatattt ttagaatatt ttaagaattt cacaagctat tccctcaaat 1440
ctgagggagc tgagtaacac catcgatcat gatgtagagt gtggttatga actttatagt 1500
tgttttatat gttgctataa taaagaagtg ttctgc                          1536
```

Figure 7a

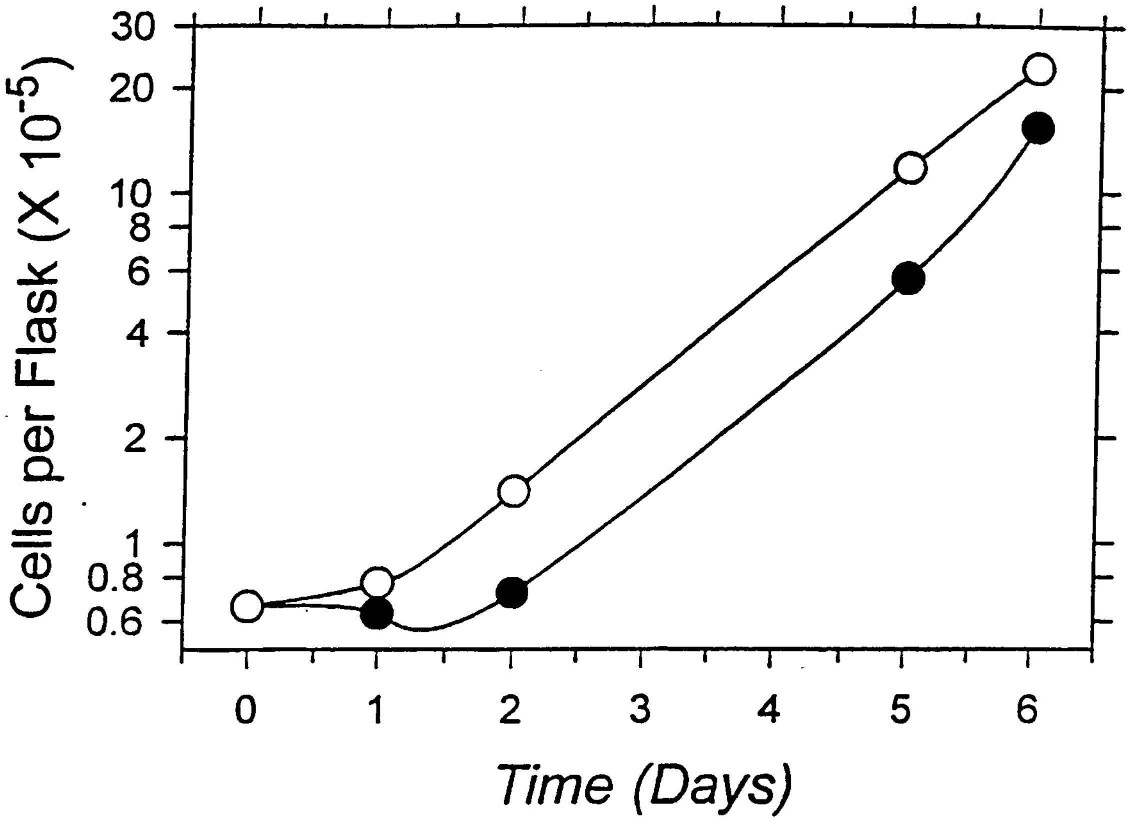


FIGURE 8

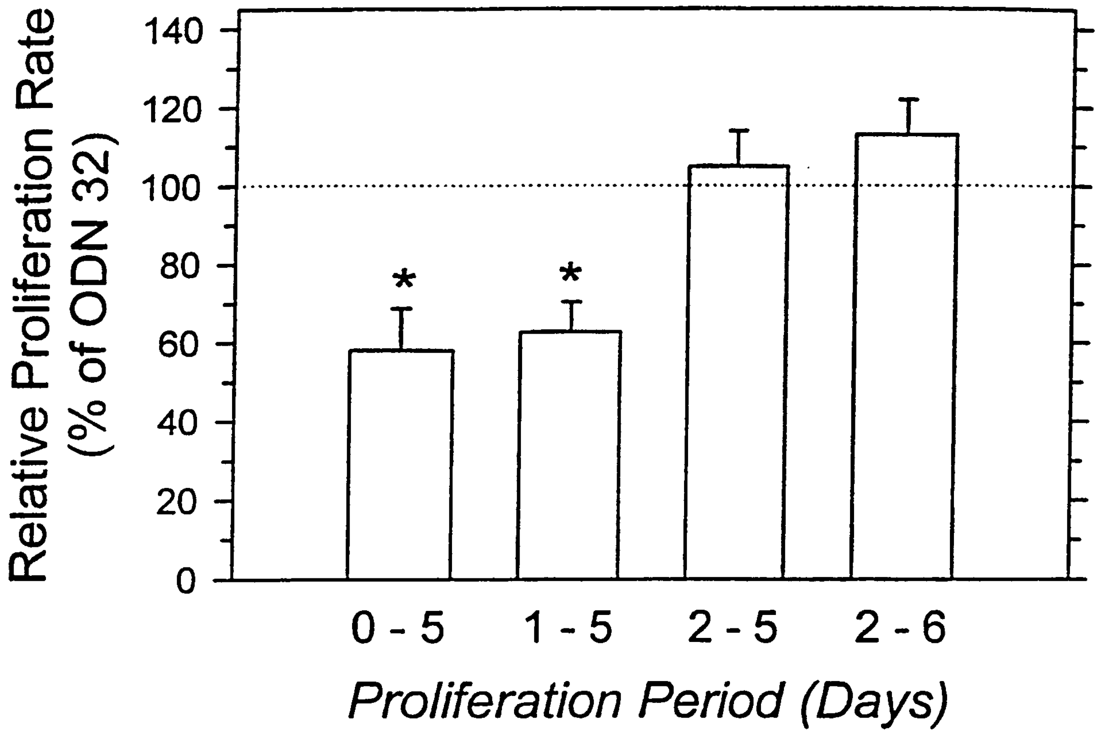
Relative Proliferation Rate
(% of ODN 32)
140
120
100
80
60
40
20
0
*
*
0 - 5
1 - 5
2 - 5
2 - 6
Proliferation Period (Days)

FIGURE 9

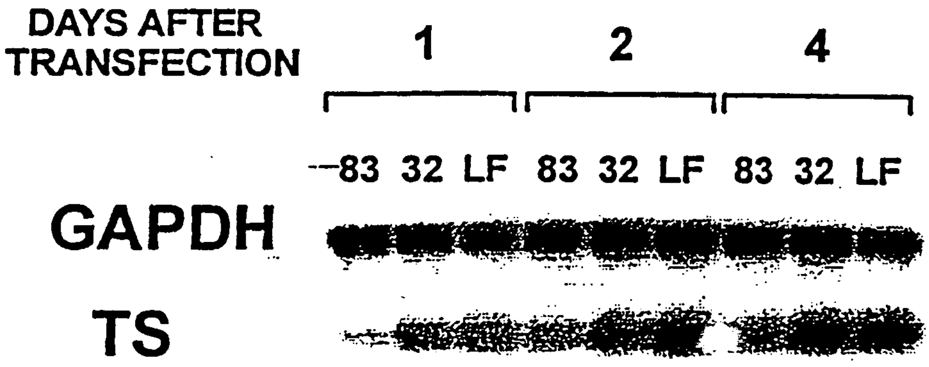


FIGURE 10

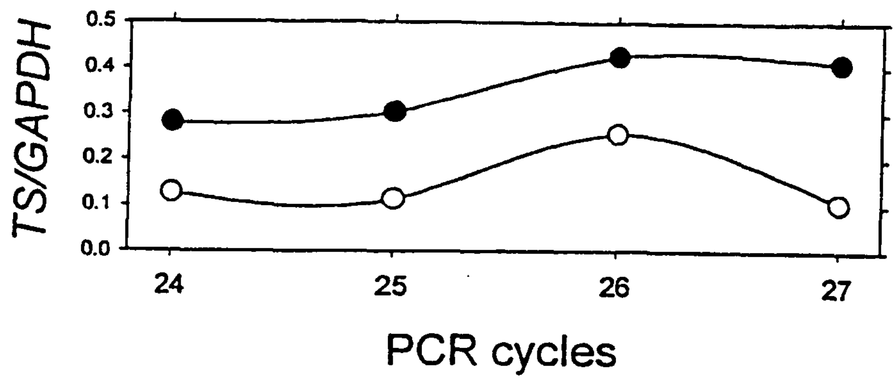


FIGURE 11

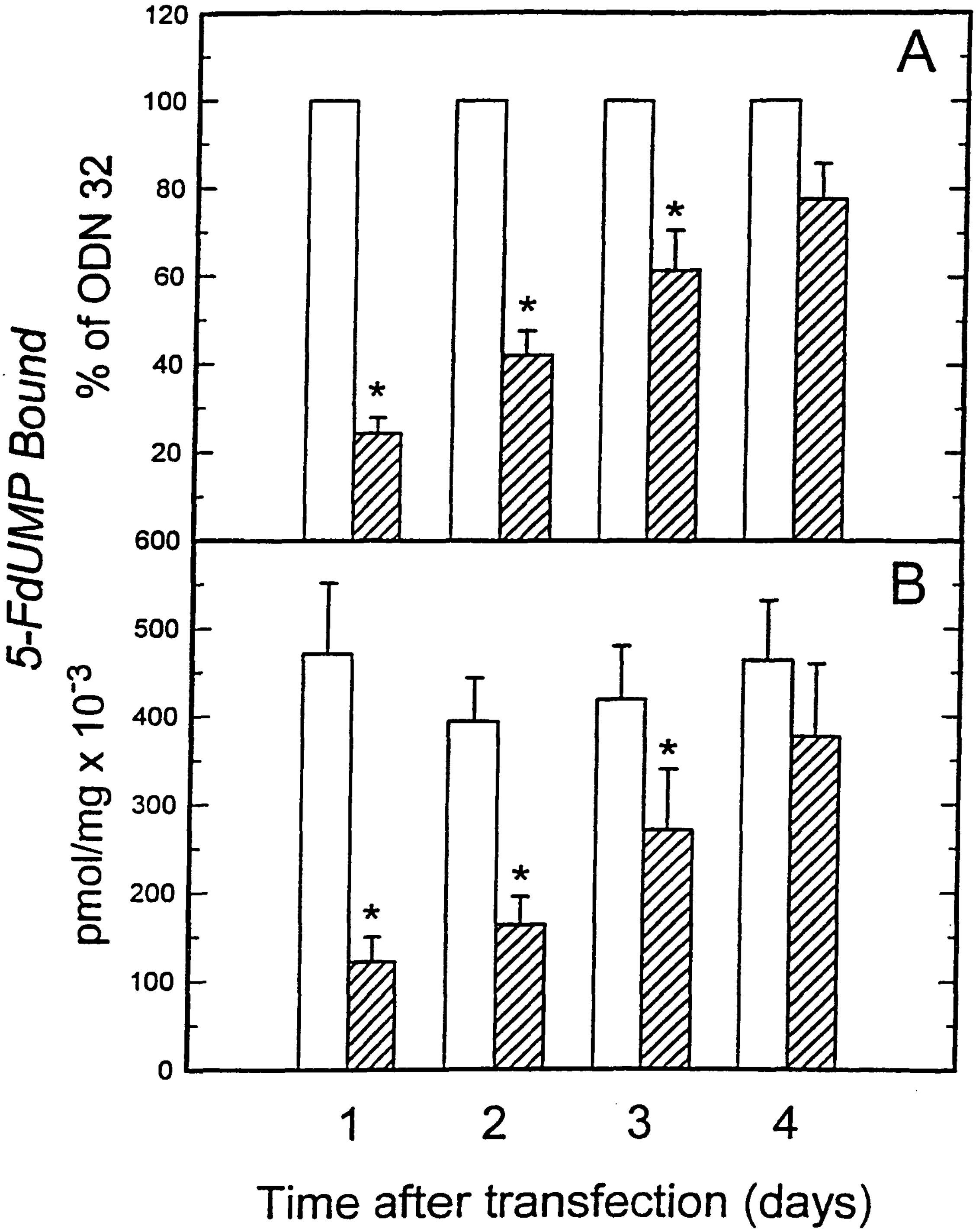

5-FdUMP Bound
% of ODN 32
pmol/mg x 10$^{-3}$
A
B
120
100
80
60
40
20
600
500
400
300
200
100
0
*
1
2
3
4
Time after transfection (days)


FIGURE 12

FIGURE 13

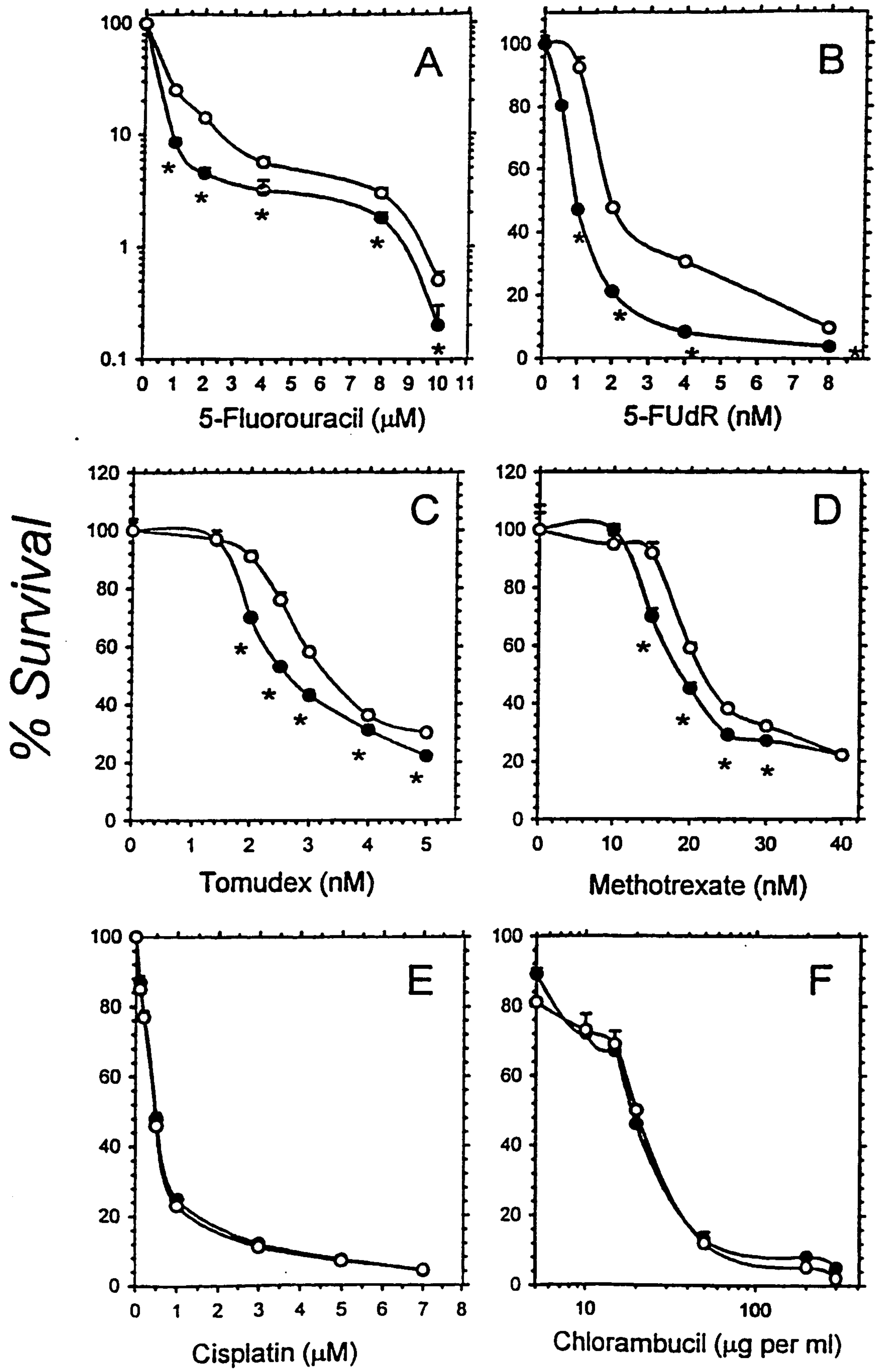
% Survival
A
5-Fluorouracil (µM)
B
5-FUdR (nM)
C
Tomudex (nM)
D
Methotrexate (nM)
E
Cisplatin (µM)
F
Chlorambucil (µg per ml)

SEQUENCE LISTING

(1) GENERAL INFORMATION:

(i) APPLICANT:

(A) NAME: Zeneca Ltd

(B) STREET: 15 Stanhope Gate
(C) CITY: London
(D) STATE: Greater London
(E) COUNTRY: England
(F) POSTAL CODE (ZIP): W1Y 6LN
(G) TELEPHONE: 0171 304 5000
(H) TELEFAX: 0171 304 5151
(I) TELEX: 0171 834 2042

(ii) TITLE OF INVENTION: COMPOUNDS
(iii) NUMBER OF SEQUENCES: 9
(iv) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(vi) PRIOR APPLICATION DATA:

(A) APPLICATION NUMBER: GB 9720107.3
(B) FILING DATE: 23-SEP-1997

(vi) PRIOR APPLICATION DATA:

(A) APPLICATION NUMBER: GB 9722012.3
(B) FILING DATE: 17-OCT-1997

(vi) PRIOR APPLICATION DATA:

(A) APPLICATION NUMBER: GB 9812140.3
(B) FILING DATE: 06-JUN-1998

(2) INFORMATION FOR SEQ ID NO: 1:

(i) SEQUENCE CHARACTERISTICS:

(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO: 1:
TTAAGGATGT TGCCACTGGC 20

(2) INFORMATION FOR SEQ ID NO: 2:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: other nucleic acid
(xi) SEQUENCE DESCRIPTION: SEQ ID NO: 2:
AATGGCTGTT TAGGGTGCTT 20

(2) INFORMATION FOR SEQ ID NO: 3:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: other nucleic acid
(xi) SEQUENCE DESCRIPTION: SEQ ID NO: 3:
TGTGGCCGGC TCGGAGCTGC 20

(2) INFORMATION FOR SEQ ID NO: 4:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: other nucleic acid
(xi) SEQUENCE DESCRIPTION: SEQ ID NO: 4:
GCGCCATGCC TGTGGCCGGC 20

(2) INFORMATION FOR SEQ ID NO: 5:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: other nucleic acid
(xi) SEQUENCE DESCRIPTION: SEQ ID NO: 5:
GCGCCATGCC TGTGGCCGGC 20

(2) INFORMATION FOR SEQ ID NO: 6:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: other nucleic acid
(xi) SEQUENCE DESCRIPTION: SEQ ID NO: 6:
CCCGCCCGCC GCGCCATGCC 20

(2) INFORMATION FOR SEQ ID NO: 7:

(i) SEQUENCE CHARACTERISTICS:

(A) LENGTH: 20 base pairs
(B) TYPE: nucleic acid

(C) STRANDEDNESS: single
(D) TOPOLOGY: linear

(ii) MOLECULE TYPE: other nucleic acid
(xi) SEQUENCE DESCRIPTION: SEQ ID NO: 7:
ATGCGCCAAC GGTTCCTAAA 20

(2) INFORMATION FOR SEQ ID NO: 8:

(i) SEQUENCE CHARACTERISTICS:

(A) LENGTH: 30 base pairs
(B) TYPE: nucleic acid
(C) STRANDEDNESS: single
(D) TOPOLOGY: linear

(ii) MOLECULE TYPE: other nucleic acid
(xi) SEQUENCE DESCRIPTION: SEQ ID NO: 8:
UGUGGCCGGC UCGGAGCUGC CGCGCCGGCC 30

(2) INFORMATION FOR SEQ ID NO: 9:

(i) SEQUENCE CHARACTERISTICS:

(A) LENGTH: 30 base pairs
(B) TYPE: nucleic acid
(C) STRANDEDNESS: single
(D) TOPOLOGY: linear

(ii) MOLECULE TYPE: other nucleic acid
(xi) SEQUENCE DESCRIPTION: SEQ ID NO: 9:
GCUACAGCCU GAGAGAUGAA UUCCCUCUGC 30

SEQUENCE LISTING

<110> ISIS Pharmaceuticals Sarissa Inc.

<120> Antisense Oligonucleotides Against Thymidylate Synthase

<130> 753-109EP

<140> 98942944.4
<141> 1998-09-17

<160> 21

<170> FastSEQ for Windows Version 4.0

<210> 1
<211> 20
<212> DNA
<213> Artificial Sequence

<220>
<223> Description of Artificial Sequence: Synthetic Oligonucleotide

<400> 1
gccagtggca acatccttaa 20

<210> 2
<211> 20
<212> DNA
<213> Artificial Sequence

<220>
<223> Description of Artificial sequence: Synthetic Oligonucleotide

<400> 2
aagcacccta aacagccatt 20

<210> 3
<211> 20
<212> DNA
<213> Artificial Sequence

<220>
<223> Description of Artificial Sequence: Synthetic Oligonucleotide

<400> 3
gcagctccga gccggccaca 20

<210> 4
<211> 20
<212> DNA
<213> Artificial Sequence

<220>
<223> Description of Artificial Sequence: Synthetic Oligonucleotide

<400> 4
gccggccaca ggcatggcgc 20

<210> 5

<400> 5
000

<210> 6
<211> 20
<212> DNA
<213> Artificial Sequence

<220>
<223> Description of Artificial Sequence: Synthetic Oligonucleotide

<400> 6
ggcatggcgc ggcgggcggg 20

<210> 7
<211> 20
<212> DNA
<213> Artificial Sequence

<220>

<223> Description of Artificial Sequence: Synthetic Oligonucleotide

<400> 7
atgcgccaac ggttcctaaa 20

<210> 8
<211> 30
<212> RNA
<213> Artificial Sequence

<220>
<223> Description of Artificial Sequence: Synthetic Oligonucleotide

<400> 8.
uguggccggc ucggagcugc cgcgccggcc 30

<210> 9
<211> 30
<212> RNA
<213> Artificial Sequence

<220>
<223> Description of Artificial Sequence: Synthetic Oligonucleotide

<400> 9
gcuacagccu gagagaugaa uucccucugc 30

<210> 10
<211> 19
<212> DNA
<213> Artificial Sequence

<220>
<223> Description of Artificial Sequence: Synthetic Oligonucleotide

<400> 10
ctcagctccc tcagatttg 19

<210> 11
<211> 36
<212> DNA
<213> Artificial Sequence

<220>
<223> Description of Artificial Sequence: Probe

<400> 11
ctagatgtgg ccggctcgga gctgccgcgc cggcca 36

<210> 12
<211> 36
<212> DNA
<213> Artificial Sequence

<220>
<223> Description of Artificial Sequence: Probe

<400> 12

agcttggccg gcgcggcagc tccgagccgg ccact 36

<210> 13
<211> 36
<212> DNA
<213> Artificial Sequence

<220>
<223> Description of Artificial Sequence: probe

<400> 13
ctagagctac agcctgagag atgaattccc tctgca 36

<210> 14
<211> 36
<212> DNA
<213> Artificial Sequence

<220>
<223> Description of Artificial Sequence: Probe

<400> 14
agcttgcaga gggaattcat ctctcaggct gtagct 36

<210> 15
<211> 20
<212> DNA
<213> Artificial Sequence

<220>
<223> Description of Artificial Sequence: Probe

<400> 15
ctcttcagca cgccatggat 20

<210> 16
<211> 20
<212> DNA
<213> Artificial Sequence

<220>
<223> Description of Artificial Sequence: Synthetic Oligonucleotide

<400> 16
agggtctacc tttcttgcgc 20

<210> 17
<211> 20
<212> DNA
<213> Artificial Sequence

<220>
<223> Description of Artificial Sequence: PCR primer

<400> 17
cacactttgg gagatgcaca 20

<210> 18

<211> 25
<212> DNA
<213> Artificial Sequence

<220>
<223> Description of Artificial Sequence: PCR primer

<400> 18
ctttgaaagc accctaaaca gccat 25

<210> 19
<211> 20
<212> DNA
<213> Artificial Sequence

<220>
<223> Description of Artificial Sequence: PCR primer

<400> 19
tattgggcgc ctggtcacca 20

<210> 20
<211> 20
<212> DNA
<213> Artificial Sequence

<220> .
<223> Description of Artificial Sequence: PCR primer

<400> 20
ccaccttctt gatgtcatca 20

<210> 21
<211> 1536
<212> DNA
<213> Homo sapien

<400> 21

```
ggggggggg ggaccacttg gcctgcctcc gtcccgccgc gccacttggc ctgcctccgt 60
cccgccgcgc cacttcgcct gcctccgtcc cccgcccgcc gcgccatgcc tgtggccggc 120
tcggagctgc cgcgccggcc cttgcccccc gccgcacagg agcgggacgc cgagccgcgt 180
ccgccgcacg gggagctgca gtacctgggg cagatccaac acatcctccg ctgcggcgtc 240
aggaaggacg accgcacggg caccggcacc ctgtcggtat tcggcatgca ggcgcgctac 300
agcctgagag atgaattccc tctgctgaca accaaacgtg tgttctggaa gggtgtttg 360
gaggagttgc tgtggtttat caagggatcc acaaatgcta aagagctgtc ttccaaggga 420
gtgaaaatct gggatgccaa tggatcccga gactttttgg acagcctggg attctccacc 480
agagaagaag gggacttggg cccagtttat ggcttccagt ggaggcattt tggggcagaa 540
tacagagata tggaatcaga ttattcagga caggagttg accaactgca aagagtgatt 600
gacaccatca aaaccaaccc tgacgacaga agaatcatca tgtgcgcttg gaatccaaga 660
gatcttcctc tgatggcgct gcctccatgc catgccctct gccagttcta tgtggtgaac 720
agtgagctgt cctgccagct gtaccagaga tcgggagaca tgggcctcgg tgtgcctttc 780
aacatcgcca gctacgccct gctcacgtac atgattgcgc acatcacggg cctgaagcca 840
ggtgacttta tacacacttt gggagatgca catatttacc tgaatcacat cgagccactg 900
aaaattcagc ttcagcgaga acccagacct ttcccaaagc tcaggattct tcgaaaagtt 960
gagaaaattg atgacttcaa agctgaagac tttcagattg aagggtacaa tccgatcca 1020
actattaaaa tggaaatggc tgtttagggt gctttcaaag gagcttgaag gatattgtca 1080
gtctttaggg gttgggctgg atgccgaggt aaaagttctt tttgctctaa aagaaaaagg 1140
aactaggtca aaaatctgtc cgtgacctat cagttattaa tttttaagga tgttgccact 1200
ggcaaatgta actgtgccag ttctttccat aataaaaggc tttgagttaa ctcactgagg 1260
gtatctgaca atgctgaggt tatgaacaaa gtgaggagaa tgaaatgtat gtgctcttag 1320
caaaaacatg tatgtgcatt tcaatcccac gtacttataa agaaggttgg tgaatttcac 1380
aagctatttt tggaatattt ttagaatatt ttaagaattt cacaagctat tccctcaaat 1440
ctgagggagc tgagtaacac catcgatcat gatgtagagt gtggttatga actttatagt 1500
tgttttatat gttgctataa taaagaagtg ttctgc                           1536
```